# EUROPÄISCHE PATENTSCHRIFT

(11) **EP 0 321 841 B1**
(45) Veröffentlichungstag und Bekanntmachung des Hinweises auf die Patenterteilung: **14.04.1993**
(21) Anmeldenummer: 88120877.1
(22) Anmeldetag: 14.12.1988
(51) Int. Cl.: C07C 271/08, C08F 20/36, A61K 6/08

(54) **Ester-Urethan-(Meth)-acrylsäurederivate**
Ester urethane (met)acrylic-acid derivatives
Dérivés de (met) acrylate d'uréthane

(30) Priorität: 23.12.1987 DE 3743782
(43) Veröffentlichungstag der Anmeldung: 28.06.1989
(73) Patentinhaber: BAYER AG, 51368 Leverkusen (DE)
(72) Erfinder: Reiners, Jürgen, Dr., D-5090 Leverkusen 1 (DE); Süling, Carlhans, Dr., D-5068 Odenthal (DE); Schäfer, Walter Dr.,, D-5653 Leichlingen (DE); Müller, Hanns Peter, Dr., D-5060 Berg.-Gladbach 2 (DE); Podszun, Wolfgang, Dr., D-5000 Köln 80 (DE); Winkel, Jens, Dr., D-5000 Köln-Pesch (DE)

(56) Entgegenhaltungen:
- EP-A- 0 254 950
- DE-A- 3 710 279
- US-A- 3 748 133
- US-A- 4 420 306
- US-A- 4 554 336

## Beschreibung

Die Erfindung betrifft neue Ester-Urethan-(Meth)-acrylsäurederivate, ihre Herstellung und ihre Verwendung als monomere Komponenten für Dentalwerkstoffe.

Die Verwendung von polyfunktionellen (Meth)-acrylsäurederivaten als Komponenten für Zahnfüllungsmaterialien ist bekannt. So werden in der EP-A 0 017 936 Acrylsäureester und Meth-acrylsäureester von Pentaerythrit beschrieben. Die dort beschriebenen Monomeren ergeben in Kombination mit anorganischen Füllstoffen Dentalwerkstoffe, die einen unerwünschten Polymerisationsschrumpf aufweisen, der zu einer Spaltung zwischen Zahn- und Füllungsmaterial führt.

In der US 4 554 336 werden Urethangruppen enthaltende (Meth)-acrylsäurederivate für Adhesive im Dentalbereich beschrieben, bei denen die Urethangruppen durch einen eine (Meth)-acrylatgruppe enthalten Rest substituiert sind. Diese Verbindungen zeigen als Komponenten in Dentalmassen unzureichende Eigenschaften, insbesondere eine für die Praxis zu geringe Festigkeit.

Es wurden nun neue Ester-Urethan-(Meth)-acrylsäurederivate der Formel (I) 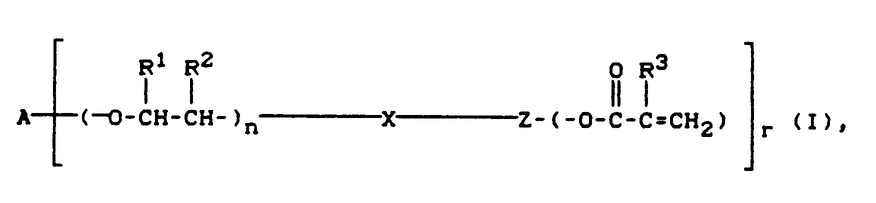
in der
- A: ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
- r: für die Anzahl der von A ausgehenden Ketten steht und eine Zahl von 2 bis 6 bedeutet,
- R¹ und R²: gleich sind und Wasserstoff oder verschieden sind und Wasserstof und Methyl bedeuten,
- n: für jede von A ausgehende Kette unabhängig eine Zahl von 0 bis 5 bedeutet,
- X: für die Gruppe 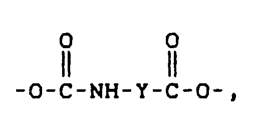 in der
- Y: einen zweiwertigen (cyclo)aliphatischen Rest mit 2 bis 15 C-Atomen, der gegebenenfalls Ester-, Ether- oder Urethangruppen enthält, bedeutet,
steht,
- Z: einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acrylatreste substituiert sein kann, bedeutet, und
- R³: für jede von A ausgehende Kette unabhängig Wasserstoff oder Methyl bedeutet,
gefunden.

Dentalwerkstoffe, bei denen von den erfindungsgemäß Ester-Urethan-(Meth)-acrylsäurederivaten ausgegangen wird, zeigen überraschenderweise einen wesentlich geringeren Polymerisationsschrumpf, höhere Verschleißfestigkeit und eine größere Festigkeit und sind daher für die Anwendung in der Praxis besonders geeignet. Besonders vorteilhaft ist, daß die Produkte aufgrund der geringen Viskosität des Basismonomers einen höheren Anteil dieses Monomeren besitzen.

Im Rahmen der vorliegenden Erfindung können die Substituenten folgende Bedeutung haben:
Ein aliphatischer Rest (A) kann ein geradkettiger oder verzweigter Kohlenwasserstoffrest mit 2 bis 20, bevorzugt 3 bis 12, Kohlenstoffatomen sein. Beispielsweise seien die folgenden aliphatischen Rest genannt:

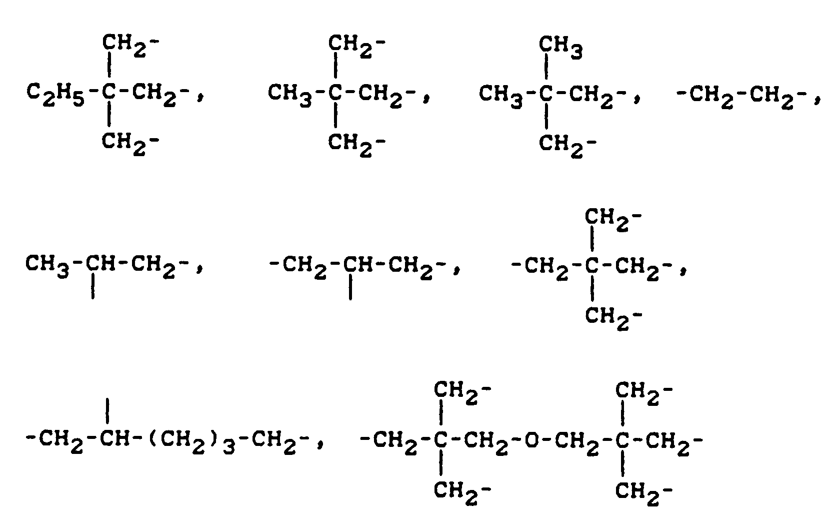

Ein aromatischer Rest (A) kann ein aromatischer Kohlenwasserstoffrest mit 6 bis 24, bevorzugt 6 bis 14, Kohlenstoffatomen sein. Beispielsweise seinen die folgenden aromatischen Reste genannt:

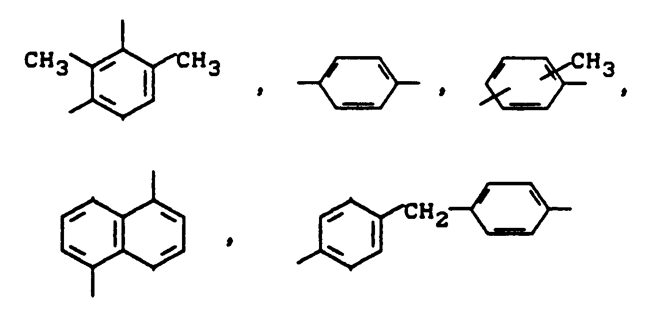

Ein araliphatischer Rest (A) kann ein Kohlenwasserstoffrest mit einem geradkettigen oder verzweigten aliphatischen und einem aromatischen Teil mit 7 bis 26 Kohlenstoffatomen bedeuten, wobei der aromatische Teil bevorzugt 6 bis 12 und der aliphatische Teil bevorzugt 1 bis 14 Kohlenstoffatome enthält. Beispielsweise seien die folgenden araliphatischen Reste genannt:

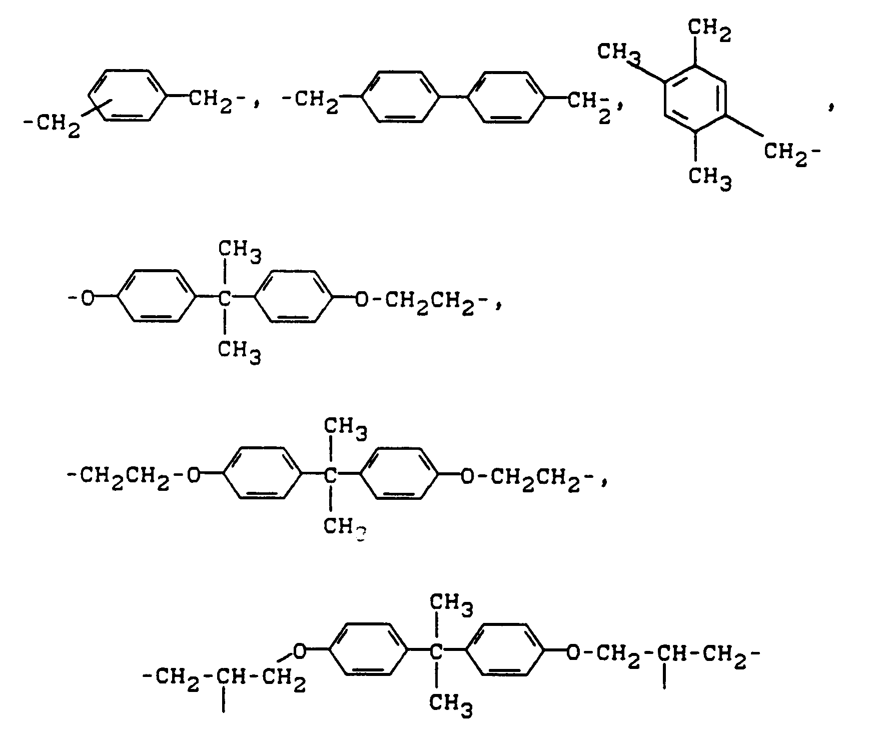

Ein cycloaliphatischer Rest (A) kann ein cyclischer Kohlenwasserstoffrest mit 6 bis 26 kohlenstoffatomen, bevorzugt 6 bis 14 Kohlenstoffatomen, sein. Beispielsweise seien die folgenden cycloaliphatischen Reste genannt:

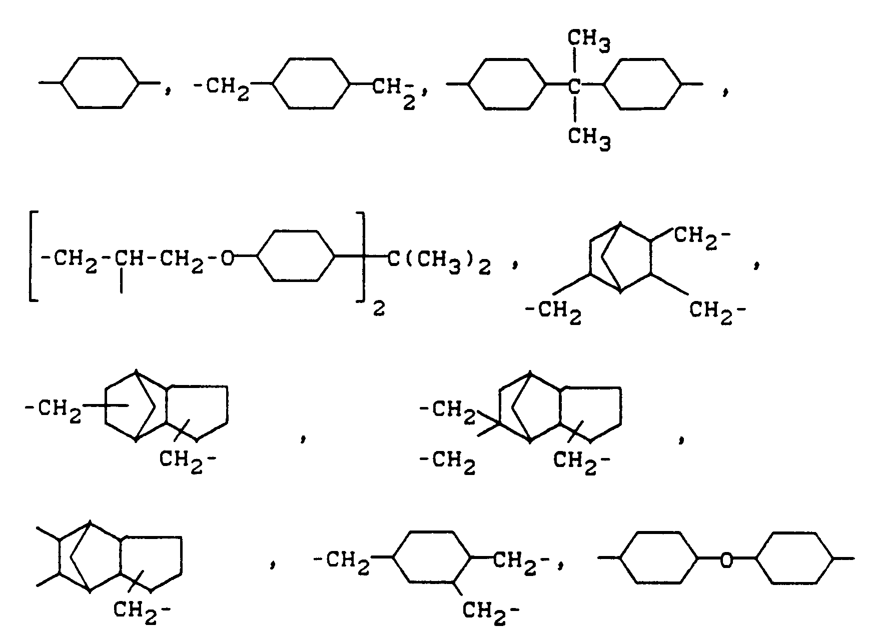

Die Reste A können, bevorzugt im aliphatischen oder cycloaliphatischen Teil, 1 oder 2, bevorzugt 1, Sauerstoffatome enthalten, so daß beispielsweise aliphatische bzw. cycloaliphatische Ether vorliegen.

Insbesondere bevorzugt seien die folgenden Reste A genannt: Ethylen, Propylen, 2,2-Bismethylen-butan-1-yl, 2,2-Bismethylen-propan-1-yl, 2,2-Bis-methylen-propan-1,3-diyl, 1,1′-Oxy-bis-[(2,2-methylen)-propan-1,3-diyl], Propan-1,2,3-triyl, 1,6-Hexamethylen, 1,4-Tetramethylen, 1,4-Phenylen, Xylylen, 1,4-Cyclohexylen, 1,4-Bismethylen-1,4-cyclohexan, 2,2-bis(1,4-phenylen)propan, 3(4), 8(9)-Bismethylen-tricyclo[5.2.1.0^{2,6}]decan und dessen Isomere, 4(5),9-Bismethylen-3,8-dimethyltricyclo[5.2.1.0^{2,6}]decan.

Insbesondere bevorzugt sind die Reste 2,2-Bismethylen-butan-1-yl, Propan-1,2,3-triyl, 2,2-Bismethylenpropan-1,3-diyl und 3(4),8(9)-Bismethylen-tricyclo[5.2.1.0^{2,6}]decan.

Als Gruppen X werden beispielsweise genannt:

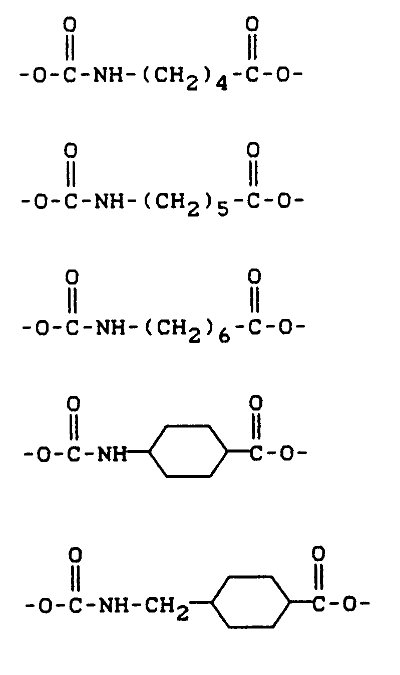

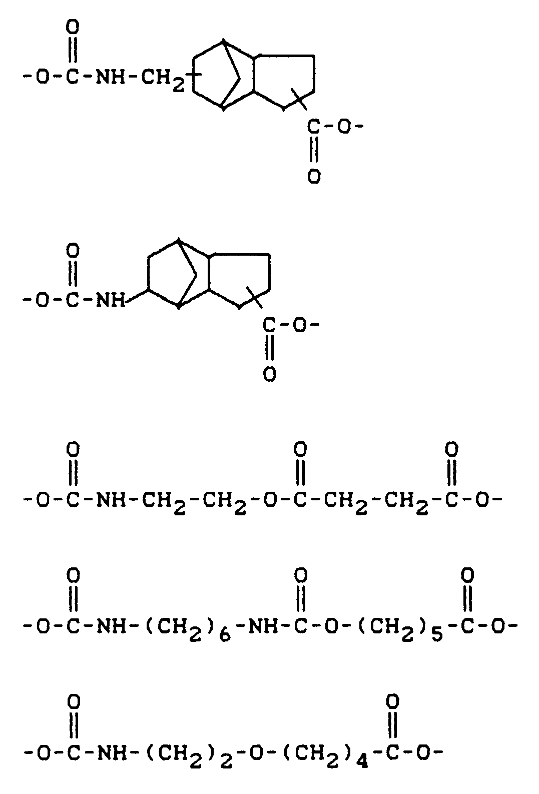

Ein zweiwertiger Kohlenwasserstoffrest (Z) kann ein geradkettiger oder verzweigter aliphatischer Kohlenwasserstoff mit 3 bis 15 Kohlenstoffatomen, bevorzugt 3 bis 10 Kohlenstoffatomen bedeuten. Der Rest Z kann gegebenenfalls 1 bis 3 Sauerstoffbrücken, bevorzugt 1 bis 2 Sauerstoffbrücken enthalten. Es ist auch möglich, daß der Rest Z durch 1 bis 4, bevorzugt 1 bis 2 (Meth)-acrylatreste substituiert ist. Beispielsweise seien die folgenden Reste genannt:

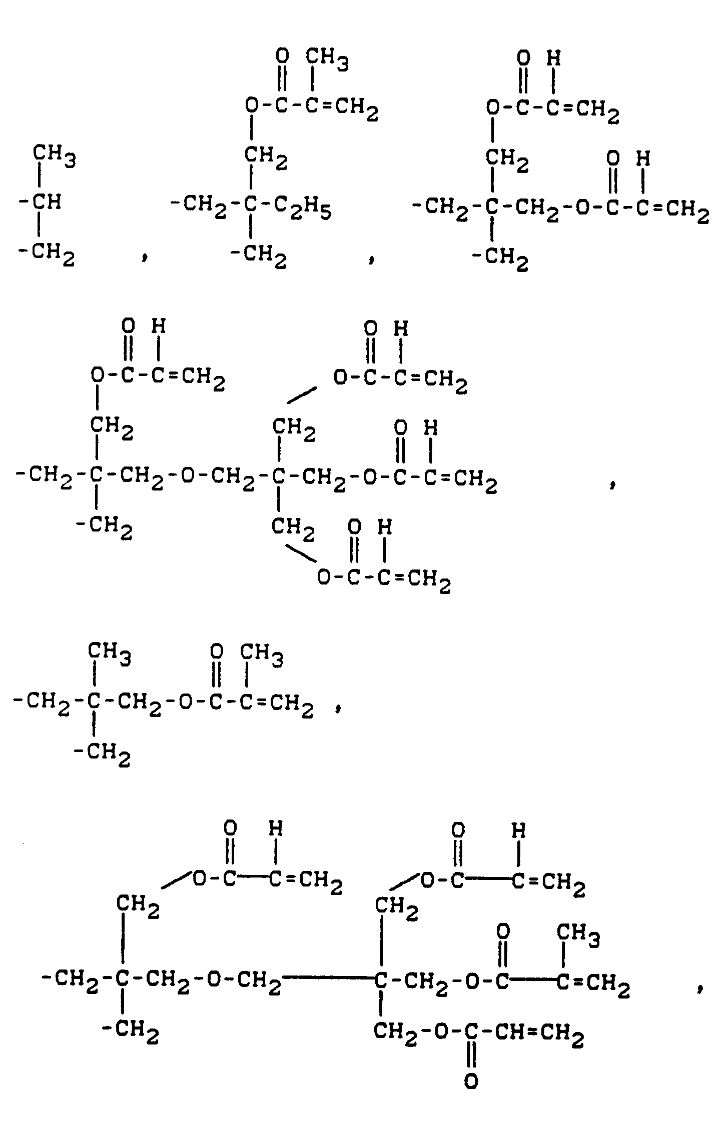

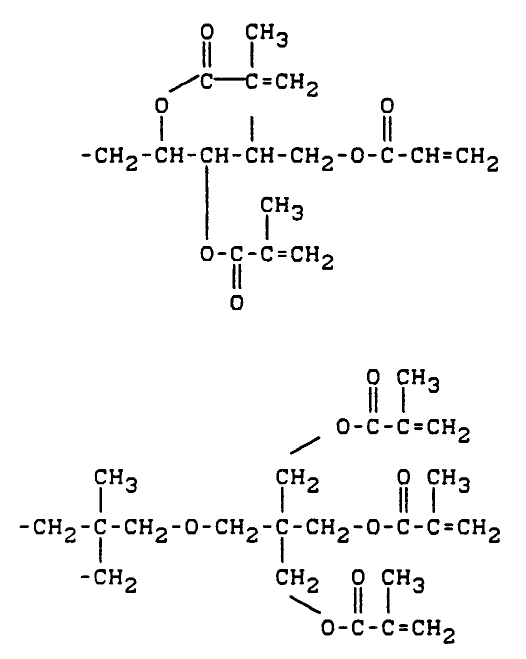

Bevorzugt werden Ester-Urethan-(Meth)-acrylsäurederivate der Formel (I), bei denen
- A: ein geradkettiger oder verzweigter, gegebenenfalls 1 bis 3 Sauerstoffbrücken enthaltender, aliphatischer Rest mit 3 bis 12 Kohlenstoffatomen, ein aromatischer Rest mit 6 bis 14 Kohlenstoffatomen, ein araliphatischer Rest mit 7 bis 26 Kohlenstoffatomen oder ein cycloaliphatischer Rest mit 6 bis 14 Kohlenstoffatomen ist,
- r: für die Anzahl der von A ausgehenden Ketten steht und eine Zahl von 2 bis 6 bedeutet,
- R¹ und R²: gleich sind und Wasserstoff oder verschieden sind und Wasserstoff und Methyl bedeuten,
- n: für jede von A ausgehende Kette unabhängig eine Zahl von 0 bis 5 bedeutet,
- X: für eine Gruppe 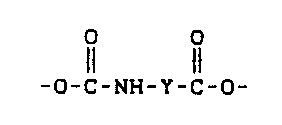 in der
- Y: einen zweiwertigen aliphatischen Rest mit 2 bis 10 Kohlenstoffatomen bedeutet,
steht,
- Z: ein zweiwertiger geradkettiger oder verzweigter aliphatischer Kohlenwasserstoff mit 3 bis 10 Kohlenstoffatomen, der gegebenenfalls 1 oder 2 Sauerstoffbrücken enthalten kann und gegebenenfalls durch 1 oder 2 (Meth)-acrylatreste substituiert sein kann, bedeutet, und
- R³: für jede von A ausgehende Kette unabhängig Wasserstoff oder Methyl bedeutet.

Insbesondere bevorzugt werden Urethangruppen enthaltende (Meth)-acrylsäurederivate der Formel (I), bei denen
- A: für den 2,2-Bismethylen-butan-1-yl-Rest, Propan-1,2,3-triyl-Rest, 2,2-Bismethylenpropan-1,3-diyl-Rest oder 3(4), 8(9)-Bismethylen-tricyclo[5.2.1.0^{2,6}]decan-Rest steht,
- r: für die Anzahl der von A ausgehenden Ketten steht und die Zahl 3 oder 4 bedeutet,
R¹ und R² gleich sind und Wasserstoff oder verschieden sind und Wasserstoff und Methyl bedeuten,
- n: für jede von A ausgehende Kette unabhängig eine Zahl von 0 bis 5 bedeutet,
- X: die Gruppe 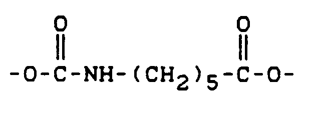 bedeutet,
- Z: einen zweiwertigen geradkettigen oder verzweigten aliphatischen Kohlenwasserstoffrest mit 3 bis 10 Kohlenstoffatomen, der gegebenenfalls 1 Sauerstoffbrücke enthalten und gegebenenfalls durch 1 (Meth)-acrylatrest substituiert sein kann, bedeutet, und
- R³: für jede von A ausgehende Kette unabhängig Wasserstoff oder Methyl bedeutet.

Beispielsweise seien die folgenden Ester-Urethan-(Meth)-acrylsäurederivate genannt:

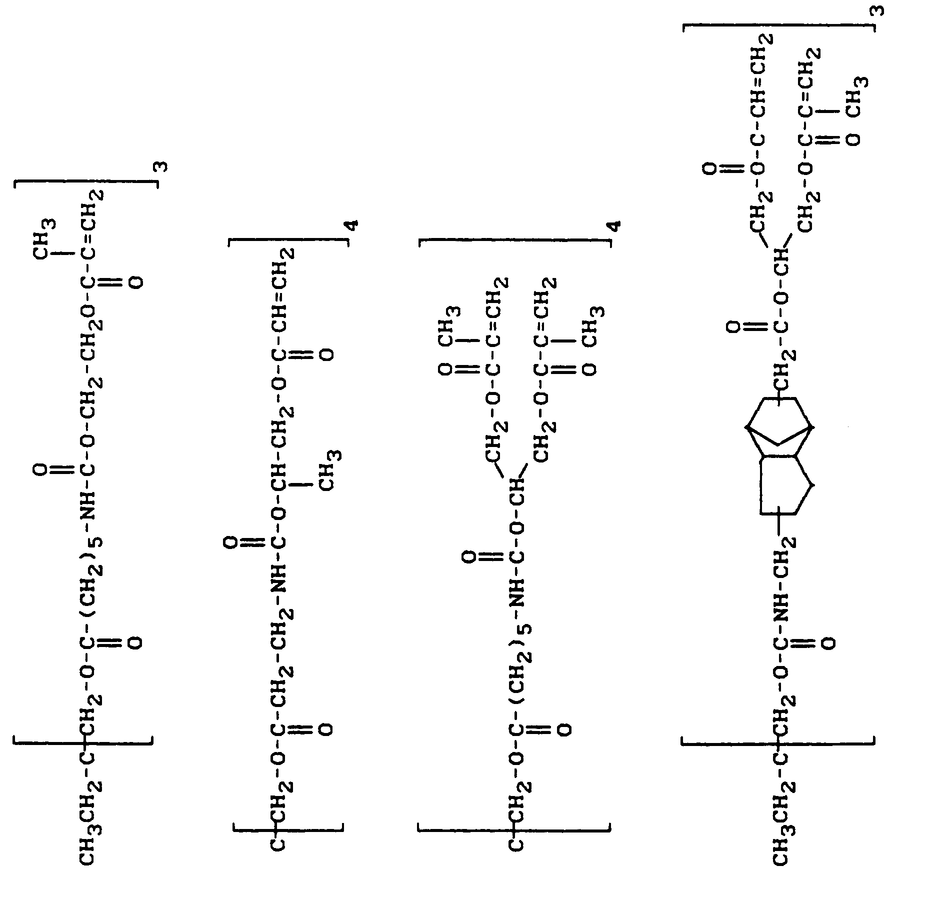

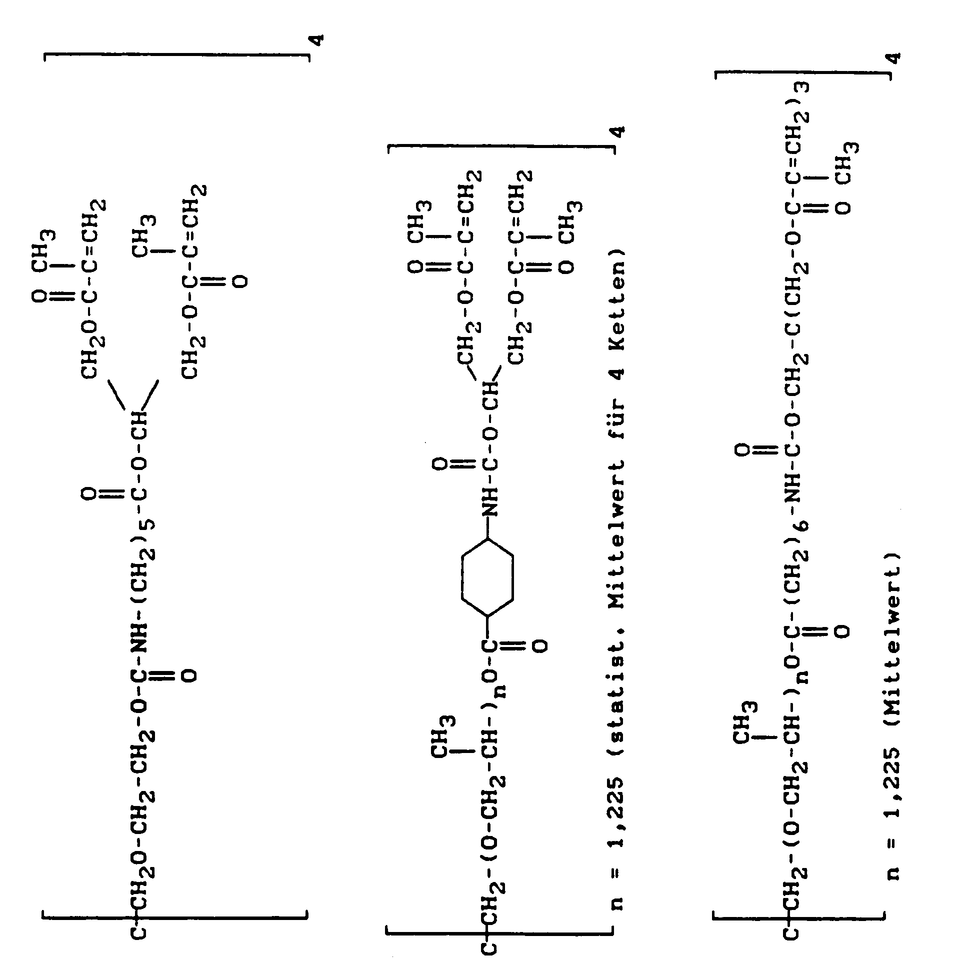

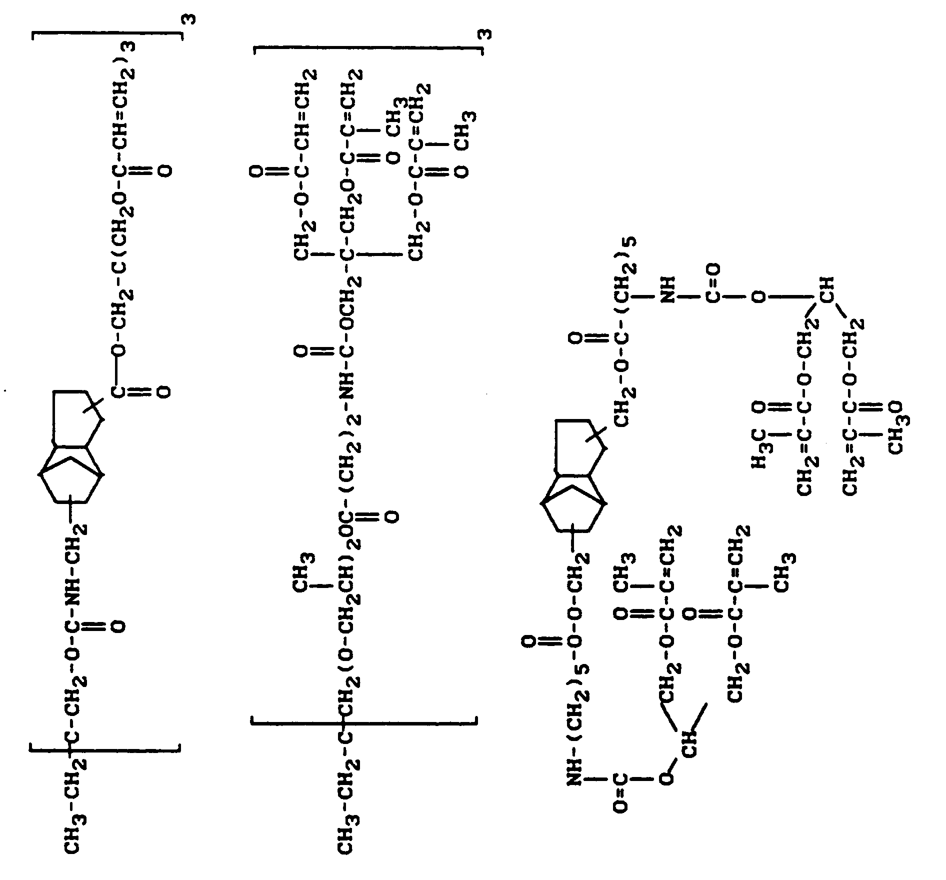

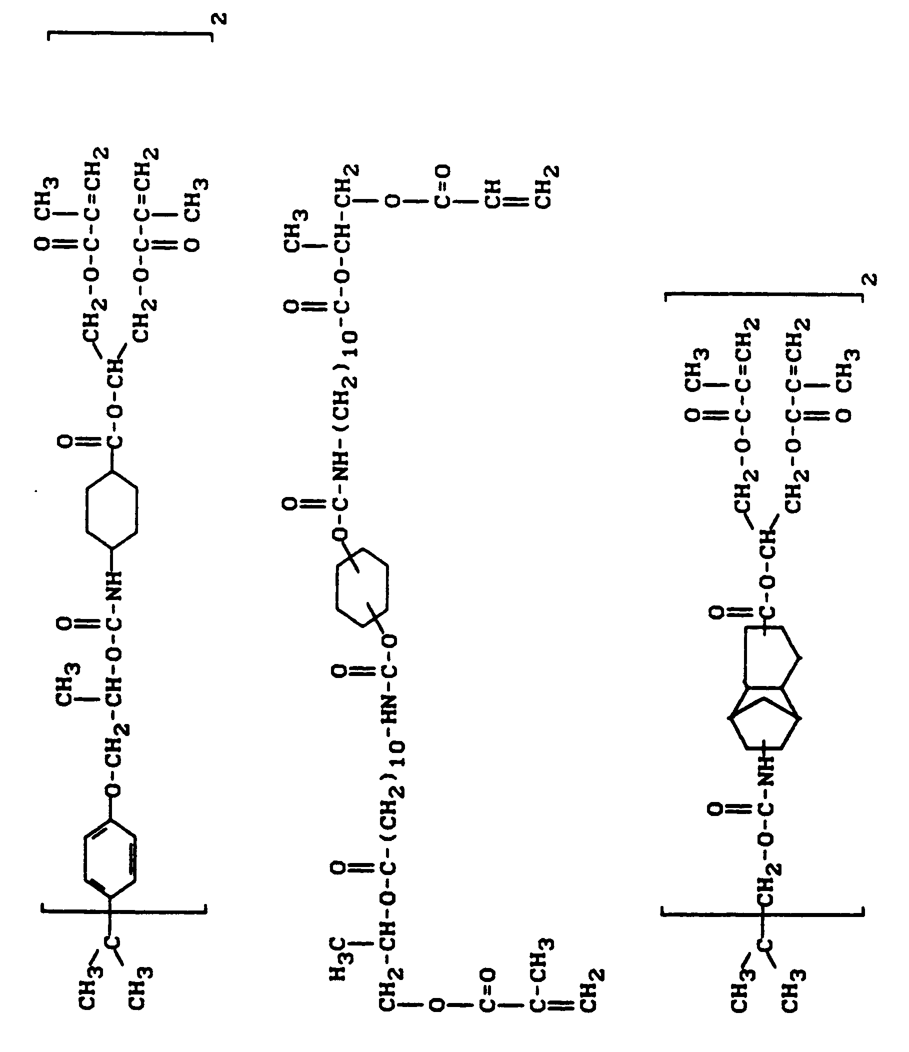

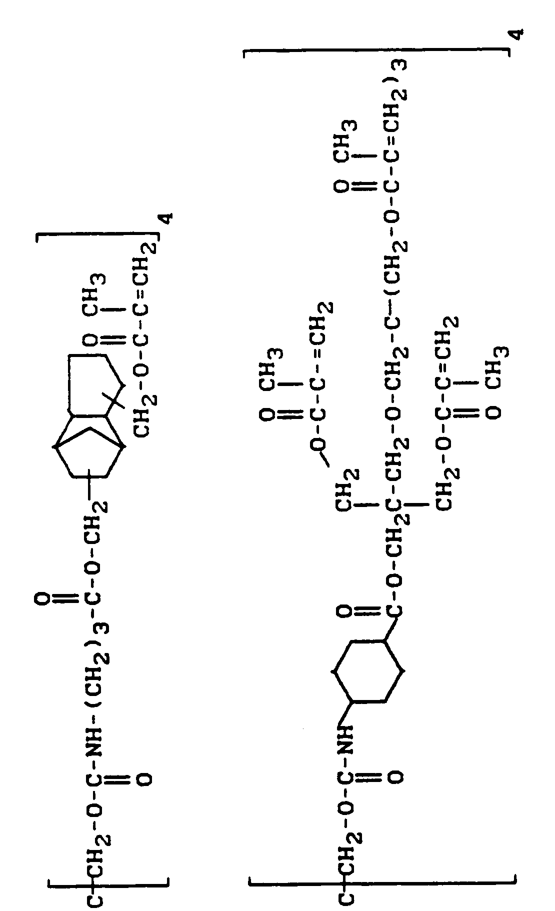

Es wurde auch ein Verfahren zur Herstellung der erfindungsgemäßen Ester-Urethan(Meth)-acrylsäurederivate der Formel (I) 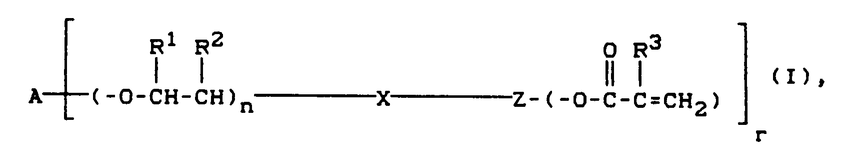
in der
- A: ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
- r: für die Anzahl der von A ausgehenden Ketten steht und eine Zahl von 2 bis 6 bedeutet,
- R¹ und R²: gleich sind und Wasserstoff oder verschieden sind und Wasserstoff und Methyl bedeuten,
- n: für jede von A ausgehende Kette unabhängig eine Zahl von 0 bis 5 bedeutet,
- X: für eine Gruppe 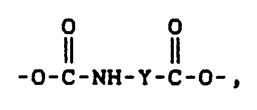 in der
- Y: einen zweiwertigen (cyclo)aliphatischen Rest mit 2 bis 15 C-Atomen, der gegebenenfalls Ester-, Ether- oder Urethangruppen enthält, bedeutet,
steht,
- Z: ein zweiwertiger geradkettiger oder verzweigten aliphatischen Kohlenwasserstoff mit 3 bis 15 Kohlenstoffatomen, der gegebenenfalls 1 bis 3 Sauerstoffbrücken enthalten kann und gegebenenfalls durch 1 bis 4 (Meth)-acrylatreste substituiert sein kann, bedeutet, und
- R³: für jede von A ausgehende Kette unabhängig Wasserstoff oder Methyl bedeutet,
dadurch gekennzeichnet, daß man den Trialkylsilylether eine Polyols der Formel (II) 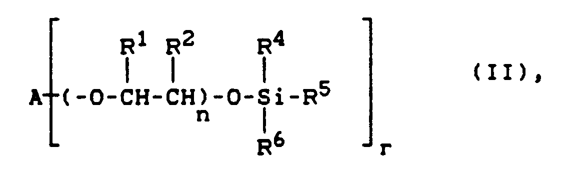
in der
A, R¹, R², n und r die obengenannte Bedeutung haben und
R⁴ bis R⁶ einen Alkylrest mit 1 bis 4 Kohlenstoffatomen bedeuten,
mit einem Isocyanatocarbonsäurechlorid der Formel (III) 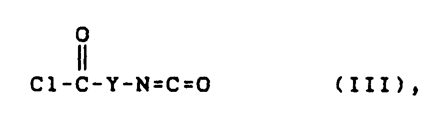
in der
- Y: die obengenannte Bedeutung hat,
im Molverhältnis von etwa 1:1, gegebenenfalls in einem inerten Lösungsmittel, umsetzt und dann den entstandenen Isocyanatoester mit einem Hydroxyalkyl(meth)acrylsäureester der Formel (IV) 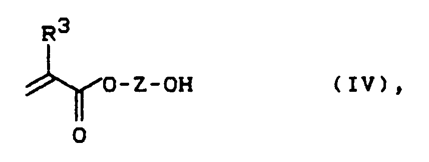
in der
R³ und Z die obengenannte Bedeutung haben,
im Molverhältnis von OH-Gruppen zu NCO-Gruppen von 0,98:1 bis 1,05:1 umsetzt,
gefunden.

Die den Trialkylsilylethern der Formel II zugrundeliegenden Polyole sind an sich bekannt (DE-A 2 931 925) bzw. handelsüblich und können beispielsweise durch Oxyalkylierung der bekannten Polyole der Formel A(OH)ᵣ, z.B. 2,2-Bishydroxymethylbutan, 2,2-Bishhydroxymethylpropan-1,3-diol, 3(4),8(9)-Bishydroxymethyl-tricyclo[5.2.1.0^{2,6}]decan usw. hergestellt werden. Durch die Herstellung bedingt, können die Polyole auch als Produkt mit variablem Oxyalkylierungsgrad vorliegen.

Die Silylether II sind in bekannter Weise, z.B. wie bei der Derivatisierung von Polyolen zu chromatographischen Zwecken, aus den entsprechenden Polyolen und Silylierungsreagenzien zugänglich. Zur Silylierung eignen sich z.B. Trialkylchlorsilane, Hexaalkyl-disilazane usw.

Isocyanatocarbonsäurechloride der Formel III sind bekannt und können durch Umsetzung der Aminocarbonsäuren mit Phosgen hergestellt werden (W. Mormann, S. Hoffmann, W. Hoffmann, Chemische Berichte 120, 285-290 (1987); DE-A 2 120 090).

(Meth)-acrylsäureester der Formel IV sind an sich bekannt und können beispielsweise durch partielle Veresterung der entsprechenden Polyole erhalten werden.

### 1. Stufe:

Die Umsetzung des Trialkylsilylethers des Polyols II mit dem Isocyanatocarbonsäurechlorid III wird im allgemeinen ohne die Verwendung von Lösungsmitteln im Temperaturbereich von 80 bis 150°C durchgeführt. Lösungsmittel werden gegebenenfalls dann eingesetzt, wenn das Isocyanatocarbonsäurechlorid ein Feststoff ist, der sich im Trialkylsilylether des Polyols nicht löst. Geeignet sind Lösungsmittel, die keinen aciden Wasserstoff enthalten, wie Toluol, Xylol, Dioxan, Acetonitril, Methylisobutylketon usw.

Die Reaktion wird unter Normaldruck oder reduziertem Druck durchgeführt.

Die Reaktanden werden in äquimolaren Mengen zur Reaktion gebracht; ein geringer Überschuß an III schadet nicht. Die Reaktion wird vorzugsweise zunächst bei Normaldruck bei 80 - 150°C durchgeführt, wobei bereits ein Teil des entstehenden Trialkylchlorsilans abdestilliert, z.B. bei Verwendung von Trimethylsilylethern und Triethylsilylethern. Um die Reaktion zu vervollständigen, wird nach etwa 2 Stunden Reaktionszeit unter Vakuum das restliche Trialkylchlorsilan abdestilliert. Bei Silylethern mit Propyl- oder Butylgruppen wird von Anfang an Vakuum angelegt.

Der Umsatz ist nahezu quantitativ und kann durch gravimetrische Bestimmung des Destillats verfolgt werden.

Die nach der 1. Stufe erhaltenen Produkte stellen Isocyanato-(cyclo)alkylester der Polyole dar, wie durch spektroskopische Untersuchung gezeigt werden kann.

### 2. Stufe:

Die Umsetzung zu den Monomeren erfolgt in einer 2. Stufe, die dadurch gekennzeichnet ist, daß man das in der 1. Stufe erhaltene Isocyanat mit Hydroxyalkyl(meth)acrylsäureestern IV umsetzt.

Dabei werden 0,98 bis 1,05 Mol IV, bezogen auf 1 Mol Isocyanatgruppen des in der 1. Stufe erhaltenen Produktes eingesetzt.

Für die 2. Stufe des erfindungsgemäßen Verfahrens werden im allgemeinen inerte Lösungsmittel verwendet. Beispielsweise seien Aceton, Chloroform, Tetrahydrofuran, Dioxan, Methylenchlorid, Toluol, Acetonitril genannt. Besonders bevorzugt sind Chloroform, Toluol, Acetonitril und Aceton.

Im allgemeinen wird auch die 2. Stufe des erfindungsgemäßen Verfahrens unter Ausschluß von Wasser durchgeführt. Besonders bevorzugt wird eine maximale Menge an Wasser unter 0,1 Gew.-%, bezogen auf die Gesamtmenge der Reaktanden.

Katalysatoren für die 2. Stufe sind im allgemeinen Metallsalze höherer Fettsäuren. Bevorzugte Katalysatoren können beispielsweise Dibutylzinndilaurat, Dibutylzinndimethoxid und Zinn-(II)-octoat sein. Als Katalysatoren können aber auch Verbindungen mit tertiären Aminogruppen, wie Triethylamin, Pyridin, 2-Methyl-pyridin, N,N-Dimethylpiperazin und N,N-Dimethyl-benzylamin verwendet werden. Es ist auch möglich, Titanverbindungen wie Tetrabutyl-titanat einzusetzen.

Im allgemeinen wird der Katalysator in einer Menge von 0,1 bis 2,5 Gew.-%, bevorzugt 0,1 bis 1,5 Gew.-%, bezogen auf die Gesamtmenge der Reaktanden eingesetzt werden.

In einer bevorzugten Ausführungsform wird die 2. Stufe des erfindungsgemäßen Verfahrens in Gegenwart eines Polymerisationsinhibitors durchgeführt. Polymerisationsinhibitoren sind an sich bekannt (Ullmanns Enzyklopädie der techn. Chemie, 4. Auflage, Verlag Chemie Weinheim, Band 8, Seiten 19-45). Beispielsweise seien 2,6-Di-tert.-butyl-4-methylphenol, Hydrochinon, Hydrochinonmonomethylether genannt.

Es ist auch möglich, als Polymerisationsinhibitor Sauerstoff, z.B. Luftsauerstoff, zu verwenden, der in das Reaktionsgemisch eingeleitet wird.

Im allgemeinen wird der Polymerisationsinhibitor in einer Menge von 0,01 bis 1,0 Gew.-%, bevorzugt von 0,1 bis 0,2 Gew.-%, eingesetzt.

Die zweite Stufe des erfindungsgemäßen Verfahrens wird im allgemeinen im Temperaturbereich von 0 bis 100°C, vorzugsweise von 30 bis 70°C, durchgeführt.

Das erfindungsgemäße Verfahren wird im allgemeinen bei Normaldruck durchgeführt. Es ist aber auch möglich, das erfindungsgemäße Verfahren bei Unter- oder Überdruck (beispielsweise im Druckbereich von 0,1 bis 10 bar) durchzuführen.

Die 2. Stufe des erfindungsgemäßen Verfahrens kann beispielsweise wie folgt durchgeführt werden:
Der (Meth)-acrylsäureester der Formel (IV) und gegebenenfalls der Polymerisationsinhibitor werden im inerten Lösungsmittel gelöst und unter Rühren zu dem gegebenenfalls gelösten Produkt aus der 1. Stufe zugetropft. Der Katalysator wird dabei einem der beiden Reaktanden zugesetzt.

Bevorzugt werden 0,98 bis 1,05 Mol des Hydroxyalkyl(meth)acrylats IV, bezogen auf 1 Mol NCO-Gruppen im Produkt der 1. Stufe eingesetzt.

Die Reaktion wird im allgemeinen bis zum vollständigen Umsatz geführt. Nach beendeter Umsetzung wird das Reaktionsprodukt durch Entfernen des Lösungsmittels isoliert. Eine vorherige Filtration oder Reinigung mit Hilfe von Adsorbentien, z.B. Aktivkohle, Bleicherde, Kieselgel oder Aluminiumoxid ist möglich.

Die nach dem erfindungsgemäßen Verfahren erhaltenen (Meth)-acrylsäurederivate stellen Monomergemische mit einer für das Verfahren typischen Molmassenvertielung dar.

Es ist auch möglich, die erste und die zweite Stufe des obengenannten Verfahrens in ihrer Reihenfolge zu vertauschen. In diesem Fall müssen die entsprechenden Trialkylsilylether des Hydroxyalkyl-(meth)acrylats mit dem Isocyanatocarbonsäurechlorid umgesetzt werden. Das dabei erhaltene Isocyanato-(meth)acrylat wird dann in der 2. Stufe mit einer dem NCO-Gehalt entsprechenden Menge des Polyols umgesetzt.

Die erste Variante des Verfahrens ist jedoch bevorzugt.

Für den erfindungsgemäßen Einsatz der neuen Ester-Urethan(meth)acrylate auf dem Dentalgebiet ist eine Auftrennung der erhaltenen Reaktionsgemische nicht erforderlich. Die Gemische selbst können in vorteilhafter Weise als Komponente von Dentalwerkstoffen, beispielsweise Zahnfüllungsmaterialien, verwendet werden.

Die erfindungsgemäßen Ester-Urethan(meth)acrylate können als Monomere für Dentalwerkstoffen verwendet werden. Als Dentalwerkstoffe seien beispielsweise Füllungsmaterialien für Zähne, Beschichtungsmittel für Zähne und Komponenten für die Herstellung von Zahnersatz, bevorzugt Kunststoffzähne, genannt. Je nach Anwendungsgebiet können Dentalwerkstoffe weitere Additive enthalten.

Für die Anwendung als Monomere für polymerisierbare Zahlfüllmassen oder Beschichtungsmittel im Dentalbereich können die erfindungsgemäßen (Meth)-acrylsäurederivate mit an sich bekannten Monomeren gemischt werden, um beispielsweise die Viskosität dem Verwendungszweck anzupassen. Hierbei werden Viskositäten im Bereich von 60 bis 10.000 mPas bevorzugt. Dieses ist dadurch erreichbar, daß man den erfindungsgemäßen Monomeren gegebenenfalls ein Comonomer niedriger Viskosität als Reaktivverdünner bzw. Lösungsmittel mischt. Die erfindungsgemäßen Verbindungen werden in der Mischung mit Comonomeren mit einem Anteil von ca. 40 bis ca. 90 Gew.-%, bevorzugt von 50 bis 80 Gew.-%, eingesetzt. Es ist ebenfalls bevorzugt, Mischungen verschiedener erfindungsgemäßer (Meth)-acrylsäurederivate einzusetzen.

Es ist auch möglich, Monomermischungen einzusetzen, die mehrere Comonomere enthalten, um die gewünschte Viskosität zu erreichen.

Beispielsweise seien die folgenden Comonomeren genannt: Glycerindi(meth)acrylat, Triethylenglykoldi(meth)acrylat, Tetraethylenglykoldi(meth)acrylat, 1,12-Dodecandioldi(meth)acrylate, 1,6-Hexandioldi(meth)acrylat, Diethylenglykol dimethacrylat, 2,2-Bis-[p-(2′-hydroxy-3′-methacryloyloxy propoxy)-phenyl]-propan, 2,2-Bis-[p-(2′-methacryloyloxyethoxy)-phenyl]-propan, Trimethylol-propan-tri-(meth)-acrylat, Bis-(meth)acryloyloxyethoxymethyl-tricyclo-[5,2,1,0^{2,6}]-decan (DE-A 29 31 925 und DE-A 29 31 926).

Insbesondere bevorzugt werden Comonomere, die bei 13 mbar einen Siedepunkt über 100°C besitzen.

Die erfindungsgemäßen polyfunktionellen Ester-Urethan(Meth)-acrylsäureester können gegebenenfalls in Mischung mit den genannten Comonomeren mit an sich bekannten Methoden zu vernetzten Polymerisaten aushärten (Am. Chem. Soc., Symp. Ser. 212, 359-371 (1983)). Für die sogenannte Redoxpolymerisation ist ein System aus einer peroxidischen Verbindung und einem Reduktionsmittel, beispielsweise auf Basis tertiärer aromatischer Amine, geeignet. Beispiele für Peroxide sind: Dibenzoylperoxid, Dilauroylperoxid und Di-4-chlorbenzoylperoxid.

Als tertiäre aromatische Amine seien beispielsweise N,N-Dimethyl-p-toluidin, Bis-(2-hydroxyethyl)-p-toluidin, Bis-(2-hydroxyethyl)-3,5-dimethylanilin und N-Methyl-N-(2-Methylcarbamoyloxypropyl)-3,5-dimethylanilin genannt. Die Konzentration des Peroxids bzw. des Amins werden vorteilhaft so gewählt, daß sie 0,1 bis 5 Gew.-%, bevorzugt 0,5 bis 3 Gew.-%, bezogen auf die Monomermischung betragen. Die peroxid- bzw. aminhaltigen Monomermischungen werden bis zur Anwendung getrennt gelagert.

Die erfindungsgemäßen Monomeren können auch durch Bestrahlung mit UV-licht oder sichtbarem Licht (beispielsweise im Wellenlängenbereich von 230 bis 650 nm) zur Polymerisation gebracht werden. Als Initiatoren für die photoinitierte Polymerisation eignen sich beispielsweise Benzil, Benzildimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwart von synergistisch wirkenden Aktivatoren, wie N,N-Dimethylaminoethylmethacrylat, Triethanolamin, 4-N,N-Dimethylaminobenzolsulfonsäurediallylamid. Die Durchführung der Photopolymerisation ist beispielsweise in der DE-A 3 135 115 beschrieben.

Neben den oben beschriebenen Initiatoren können den erfindungsgemäßen (Meth)-acrylsäure-Derivaten an sich für diesen Einsatzzweck bekannte Lichtschutzmittel und Stabilisatoren zugesetzt werden.

Lichtschutzmittel sind beispielsweise in (Gächter, Müller, Taschenbuch der Kunststoff-Additive, 2. Ausgabe, Carl Hanser Verlag) beschrieben. Beispielsweise seien die folgenden Lichtschutzmittel genannt: Cyasorb UV9®, Tinuvin P®, Tinuvin 770®, Tinuvin 622®, Tinuvin 765®.

Stabilisatoren sind beispielsweise in (Ullmanns Encylconädie der technischen Chemie, 4. Auflage, Bd. 8) beschrieben. Beispielsweise seien die folgenden Stabilisatoren genannt: 2,6-Di-tert.-butylphenol, 2,6-Di-tert.-butyl-4-methylphenol, 2,6-Di-octadecyl-4-methyl-phenol, 1,1′-Methylen-bis(naphthol-2) u.a.

Die Lichtschutzmittel und die Stabilisatoren können jeweils in einer Menge von 0,01 bis 0,5 Gew.-Teilen, bezogen auf 100 Gew.-Teile der Monomermischung, eingesetzt werden.

Die Monomermischungen können ohne Zusatz von Füllstoffen als Beschichtungsmittel (Zahnlacke) eingesetzte werden.

Bei der Verwendung als Zahnfüllmassen setzt man den erhaltenen Monomermischungen im allgemeinen Füllstoffe zu. Um einen hohen Füllgrad erreichen zu können, sind Monomermischungen, die eine Viskosität im Bereich von 60 bis 10.000 mPas besitzen, besonders vorteilhaft.

Vorzugsweise mischt man den erfindungsgemäßen (Meth)acrylsäurederivaten anorganische Füllstoffe bei.Beispielsweise seien Bergkristall, Kristoballit, Quarzglas, hochdisperse Kieselsäure, Aluminiumoxid und Glaskeramiken, beispielsweise Lanthan und Zirkon enthaltende Glaskeramiken (DE-A 23 47 591) genannt. Die anorganischen Füllstoffe werden zur Verbesserung des Verbunds zur Polymermatrix des Polymethacrylats, vorzugsweise mit einem Haftvermittler vorbehandelt. Die Haftvermittlung kann beispielsweise durch eine Behandlung mit Organosiliciumverbindungen erreicht werden (Progress in Organic Coatings 11, 297-308 (1983)). Bevorzugt wird 3-Methacryloyloxypropyl-trimethoxysilan eingesetzt. Die Füllstoffe für die erfindungsgemäßen Zahnfüllmassen weisen im allgemeinen einen mittleren Teilchendurchmesser von 0,01 bis 100 µm, vorzugsweise von 0,03 bis 50 µm, besonders bevorzugt von 0,03 bis 5 µm auf. Es kann auch vorteilhaft sein, mehrere Füllstoffe nebeneinander einzusetzen, die einen voneinander verschiedenen Teilchendurchmesser und/oder einen unterschiedlichen Silangehalt besitzen.

Der Füllstoffanteil in der Zahnfüllmasse beträgt im allgemeinen 5 bis 85 Gew.-%, vorzugsweise 50 bis 80 Gew.-%.

Für die Herstellung der Zahnfüllmassen werden die Komponenten unter Verwendung handelsüblicher Knetmaschinen vermischt.

Der Anteil der erfindungsgemäßen Ester-Urethan(meth)acrylate in den Zahnfüllmassen beträgt im allgemeinen 5 bis 40 Gew.-% bezogen auf die Füllmasse.

Die erfindungsgemäßen Ester-Urethan-(Meth)-Acrylsäure-Derivate können auch also Komponenten bei der Herstellung von Zahnersatz eingesetzt werden.

Dabei werden die erfindungsgemäßen Monomeren mit die üblicherweise verwendeten, an sich bekannten Bestandteilen kombiniert. Vorzugsweise werden die Monomeren im Gemisch mit Alkylmethacrylaten, wie Methylmethacrylat eingesetzt. Es können auch zusätzlich an sich bekannte Perpolymerisate zugesetzt werden. Zur Einstellung der Zahnfabe können bekannte anorganische und organische Farbpigmente und Trübungsmittel zugesetzt werden. Auch die Anwendung von Stabilisatoren und Lichtschutzmitteln ist möglich.

Die Kunststoffzähne werden durch radikalische Polymerisation der Dentalmassen unter Formgebung hergestellt.

Die Verarbeitung ist sowohl durch Injektionsverfahren als auch durch Prägeverfahren möglich und erfolgt im allgemeinen nach den üblichen Herstellungsmethoden für Zähne auf Basis von Poly(methylmethacrylat), z.B. durch thermische Polymerisation unter Verwendung von an sich bekannten Polymerisationsinitiatoren, beisielsweise auf Basis von Peroxiden und Azoverbindungen, wie Dibenzoylperoxid, Dilauroylperoxid, Cyclohexylpercarbonat, Azobisisobuttersäurenitril. Gut geeignet sind auch Mischungen von Polymerisationsinitiatoren mit unterschiedlichen Zerfallstemperaturen.

Die aus den erfindungsgemäßen Ester-Urethan-(Meth)acrylsäureestern hergestellten Dentalwerkstoffe zeichnen sich durch hohe Widerstandsfähigkeit gegenüber mechanischer Beanspruchung une eine hohe Abrasionsbeständigkeit aus. Die Abrasionsbeständigkeit wurde nach dem in der Literatur beschriebenen in vitro-Abriebtest ermittelt (J. Thiemann, W. Finger, B. Alker, M. Bock, IADR Paper No. 650 (1986)).

### Herstellungsbeispiele

### Beispiel 1

### Tetrakis-(trimethylsilyloxymethyl)methan

Zu einer Suspension von 136 g (1 Mol) Pentaerythrit in 700 ml Toluol werden bei Raumtemperatur 163,2 g (1,5 Mol) Trimethylchlorsilan und 242,2 g (1,5 Mol) Hexamethyldisilazan langsam zugetropft. Man läßt bei Raumtemperatur mehrere Stunden rühren. Das Produktgemisch wird einer fraktionierten Destillation unterworfen:
Kp (0,3 mm Hg): 96-98°C
Ausbeute: 331 g = 78 %
Analyse durch G.L.C.: mindestens 98%

### Beispiel 2

### Herstellung des Isocyanato-esters

116,7 g (0,275 Mol) Tetrakis-(trimethylsilyloxymethyl)methan und 192,8 g (1,099 Mol) Isocyanatocapronsäurechlorid werden bei 90°C und 250 mbar 7 Stunden gerührt. Dann wird noch 15 Stunden bei 110°C und 250 mbar nachgerührt.

Das bei der Reaktion gebildete Trimethylchlorsilan (isoliert werden 110 g) wird in eine gekühlte Vorlage destilliert und kann zur Herstellung des Silylethers (Beispiel 1) wiederverwendet werden.

Das bei der Destillation als Rückstand verbleibende Produkt wird evakuiert, bis Gewichtskonstanz erreicht ist. Das Produkt ist eine gelbliche Flüssigkeit.
Ausbeute: 191 g
NCO-Gehalt: 23,4 %

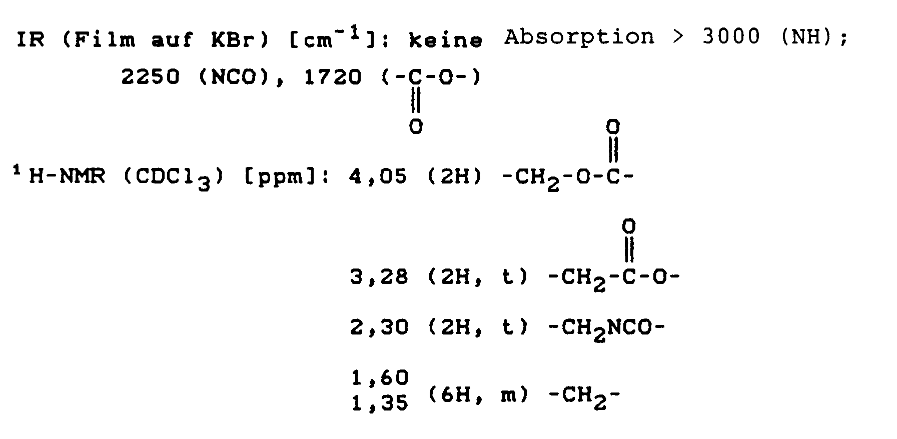

### Beispiel 3

### Herstellung eines erfindungsgemäßen Ester-Urethan-Methacrylats

191 g des in Beispeil 2 erhaltenen Polyisocyanats werden in 500 ml Chloroform gelöst. Nach Zugabe von 0,26 g 2,6-Di-tert.-butyl-4-methyl-phenol und 0,3 g Zinn-II-octoat werden bei Raumtemperatur 241,4 g (1,06 Mol) Glycerindimethacrylat (Gemisch von 1,2- und 1,3-Diester) zugegeben.

Man erhöht die Temperatur auf 60°C und rührt bei dieser Temperatur bis zum vollständigen Umsatz der NCO-Gruppen. Danach wird das Lösungsmittel im Vakuum entfernt.

IR-Spektrum (Film auf KBr) [cm⁻¹]:
3400 (N-H), 1720 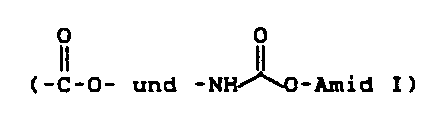
1600 (C=C), 1520 (Amid II)
Ausbeute: quantitativ

### Anwendungsbeispiele

### Beispiel 4

### Herstellung eines lichtaktivierten Beschichtungsmittels (Sealer)

In einer Mischung aus 40 Gew.-Teilen Neopentylglykoldiacrylat und 60 Gew.-Teilen Monomer aus Beispiel 3 werden 0,5 Gew.-% N,N-Diallyl-p-dimethylaminobenzolsulfonsäureamid, 0,2 Gew.-% Campherchinon und 0,125 Gew.-% Benzildimethylketal gelöst.

Beim Bestrahlen mit einer handelsüblichen Dentallampe (Translux, Fa. Kulzer) härtet die Flüssigkeit zu einem festen Kunststoff aus.

| | | |
|---|---|---|
| Biegefestigkeit | (DIN 13 922) | 85 MPa |
| Biegemodul | (DIN 13 922) | 1830 MPa |
| Wasseraufnahme | | 1,7 mg/cm² |

### Beispiel 5

### Herstellung eines lichtaktivierten Sealers

In einer Mischung aus 35 Gew.-Teilen Neopentylglykoldimethacrylat und 65 Gew.-Teilen des Monomers aus Beispiel 3 werden 0,5 Gew.- % N,N-Diallyl-p-dimethylaminobenzolsulfonsäureamid, 0,2 Gew.- % Campherchinon und 0,125 Gew.-% Benzildimethylketal gelöst.

Der nach der Bestrahlung mit einer handelsüblichen Dentallampe (60 Sek., sichtbares Licht) erhaltene Formkörper besitzt folgende Eigenschaften:

| | |
|---|---|
| Biegefestigkeit | 80 MPa |
| Biegemodul | 1690 MPa |
| Wasseraufnahme | 2,0 mg/cm² |
| Abrasionstest* | 103 ±1 % |

| | |
|---|---|
| * bezogen auf den Abrieb eines als Standard verwendeten Füllungsmaterials (Estic microfill) mit 100 %. | |

## Patentansprüche (Patentansprüche für folgende(n) Vertragsstaat(en): AT, BE, CH, DE, FR, GB, IT, LI, NL, SE)

1. Polyfunktionelle Ester-Urethan-(Meth)Acrylsäure-Derivate der Formel (I) 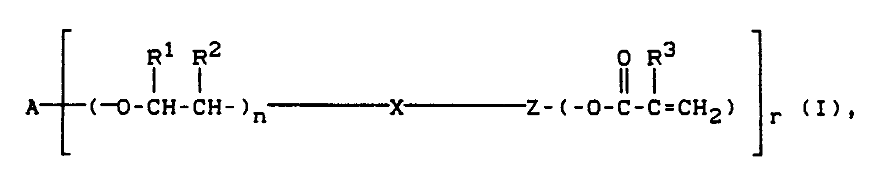 in der
A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
R¹ und R² gleich sind und Wasserstoff oder verschieden sind und Wasserstof und Methyl bedeuten,
n für jede von A ausgehende Kette unabhängig eine Zahl von 0 bis 5 bedeutet,
X für die Gruppe 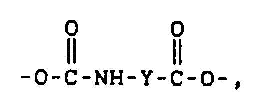 in der
Y einen zweiwertigen (cyclo)aliphatischen Rest mit 2 bis 15 Kohlenstoffatomen bedeutet, der gegebenenfalls Ester-, Ether-oder Urethangruppen enthält, bedeutet,
steht,
Z einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acrylatrest substituiert sein kann, bedeutet, und
R³ für jede von A ausgehende Kette unabhängig Wasserstoff oder Methyl bedeutet.

2. Polyfunktionelle Ester-Urethan-(Meth)-acrylsäurederivate nach Anspruch 1,
worin
A ein geradkettiger oder verzweigter, gegebenenfalls 1 bis 3 Sauerstoffbrücken enthaltender, aliphatscher Rest mit 3 bis 12 Kohlenstoffatomen, ein aromatischer Rest mit 6 bis 14 Kohlenstoffatomen, ein araliphatischer Rest mit 7 bis 26 Kohlenstoffatomen oder ein cycloaliphatischer Rest mit 6 bis 14 Kohlenstoffatomen ist,
r für die Anzahl der von A ausgehenden Ketten steht und eine Zahl von 2 bis 6 bedeutet,
R¹ und R² gleich sind und Wasserstoff oder verschieden sind und Wasserstoff und Methyl bedeuten,
n für jede von A ausgehende Kette unabhängig eine Zahl von 0 bis 5 bedeutet,
X für eine Gruppe 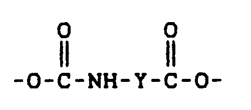 in der
Y einen Alkylen-Rest mit 2 bis 10 Kohlenstoffatomen bedeutet,
steht,
Z ein zweiwertiger geradkettiger oder verzweigter aliphatischer Kohlenwasserstoff mit 3 bis 10 Kohlenstoffatomen, der gegebenenfalls 1 oder 2 Sauerstoffbrücken enthalten kann und gegebenenfalls durch 1 oder 2 (Meth)-acrylatreste substituiert sein kann, bedeutet, und
R³ für jede von A ausgehende Kette unabhängig Wasserstoff oder Methyl bedeutet.

3. Polyfunktionelle Ester-Urethan-(Meth)acrylsäurederivate nach den Ansprüchen 1 und 2,
worin
A für den 2,2-Bismethylen-butan-1-yl-Rest, Propan-1,2,3-triyl-Rest, 2,2-Bismethylenpropan-1,3-diyl-Rest oder 3(4), 8(9)-Bismethylen-tricyclo[5.2.1.0^{2,6}]decan-Rest steht,
r für die Anzahl der von A ausgehenden Ketten steht und die Zahl 3 oder 4 bedeutet,
R¹ und R² gleich sind und Wasserstoff oder verschieden sind und Wasserstoff und Methyl bedeuten,
n für jede von A ausgehende Kette unabhängig eine Zahl von 0 bis 5 bedeutet,
X die Gruppe 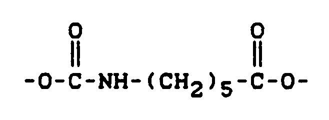 bedeutet,
Z einen zweiwertigen geradkettigen oder verzweigten aliphatischen Kohlenwasserstoffrest mit 3 bis 10 Kohlenstoffatomen, der gegebenenfalls 1 Sauerstoffbrücke enthalten und gegebenenfalls durch 1 (Meth)-acrylatrest substituiert sein kann, bedeutet, und
R³ für jede von A ausgehende Kette unabhängig Wasserstoff oder Methyl bedeutet.

4. Verfahren zur Herstellung von polyfunktionellen Ester-Urethan-(Meth)-acrylsäurederivaten der Formel (I) 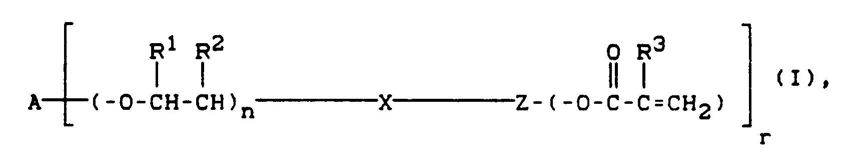 in der
A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
R¹ und R² gleich sind und Wasserstoff oder verschieden sind und Wasserstoff und Methyl bedeuten,
n für jede von A ausgehende Kette unabhängig eine Zahl von 0 bis 5 bedeutet,
X für eine Gruppe 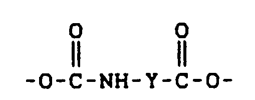 in der
Y einen zweiwertigen (cyclo)aliphatischen Rest mit 2 bis 15 Kohlenstoffatomen bedeutet, der gegebenenfalls Ester-, Ether-oder Urethangruppen enthält kann,
steht,
Z ein zweiwertiger geradkettiger oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 (Meth)-acrylatreste substituiert sein kann, bedeutet, und
R³ für jede von A ausgehende Kette unabhängig Wasserstoff oder Methyl bedeutet,
dadurch gekennzeichnet, daß man den Trialkylsilylether eine Polyols der Formel (II) 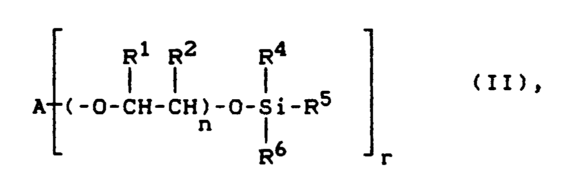 in der
A, R¹, R², n und r die obengenannte Bedeutung haben,
und
R⁴ bis R⁶ einen Alkylrest mit 1 bis 4 Kohlenstoffatomen stehen,
mit einem Isocyanatocarbonsäurechlorid der Formel (III) 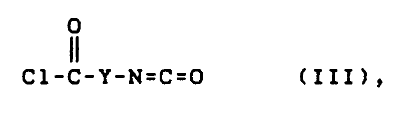 in der
Y die obengenannte Bedeutung hat,
im Molverhältnis von etwa 1:1, gegebenenfalls in einem inerten Lösungsmittel, umsetzt und dann den entstandenen Isocyanato-ester mit einem Hydroxyalkyl(meth)acrylsäureester der Formel (IV) 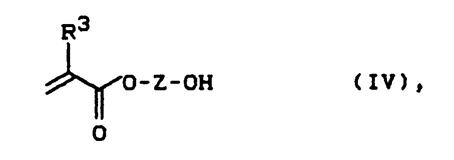 in der
R³ und Z die obengenannte Bedeutung haben,
im Molverhältnis von OH-Gruppen zu NCO-Gruppen von 0,98:1 bis 1,05:1 umsetzt.

5. Verfahren nach Anspruch 4, dadurch gekennzeichnet, daß man die Umsetzung des Silylethers mit dem Isocyanatocarbonsäurechlorid im Temperaturbereich von 80 bis 150°C durchführt.

6. Verfahren nach den Ansprüchen 4 und 5, dadurch gekennzeichnet, daß man die Umsetzung des Isocyanatoesters mit der Hydroxyverbindung im Temperaturbereich von 0 bis 100°C durchführt.

7. Polymerisat aus Ester-Urethan-(Meth)-acrylsäurederivaten der Formel 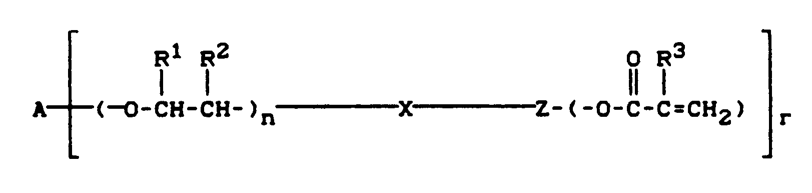 in der
A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
R¹ und R² gleich sind und Wasserstoff oder verschieden sind und Wasserstof und Methyl bedeuten,
n für jede von A ausgehende Kette unabhängig eine Zahl von 0 bis 5 bedeutet,
X für die Gruppe 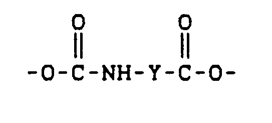 in der
Y einen zweiwertigen (cyclo)aliphatischen Rest mit 2 bis 15 Kohlenstoffatomen bedeutet, der gegebenenfalls Ester-, Ether-oder Urethangruppen enthält,
steht,
Z einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acrylatrest substituiert sein kann, bedeutet, und
R³ für jede von A ausgehende Kette unabhängig Wasserstoff oder Methyl bedeutet.

8. Verwendung von Ester-Urethan-(Meth)-acrylsäurederivaten der Formel 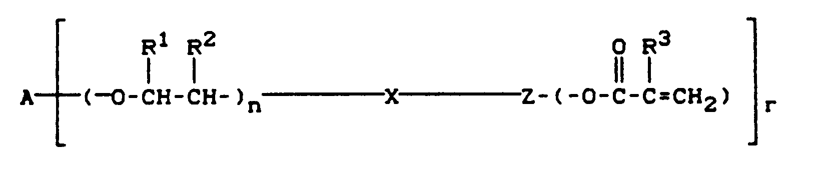 in der
A ein geradkettiger oder verzweigter, gegebenenfalls 1 bis 3 Sauerstoffbrücken enthaltender, aliphatscher Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
R¹ und R² gleich sind und Wasserstoff oder verschieden sind und Wasserstoff und Methyl bedeuten,
n für jede von A ausgehende Kette unabhängig eine Zahl von 0 bis 5 bedeutet,
X für eine Gruppe 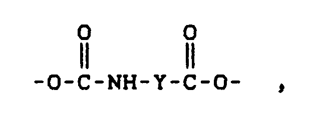 in der
Y einen zweiwertigen (cyclo)aliphatischen Rest mit 2 bis 15 Kohlenstoffatomen bedeutet, der gegebenenfalls Ester-, Ether-oder Urethangruppen enthalten kann,
steht,
Z ein zweiwertiger geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth) acrylatreste substituiert sein kann, bedeutet, und
R³ für jede von A ausgehende Kette unabhängig Wasserstoff oder Methyl bedeutet,
in Dentalwerkstoffen.

9. Verwendung nach Anspruch 8, dadurch gekennzeichnet, daß die Ester-Urethan-(Meth)-acrylsäurederivate in Zahnfüllmassen eingesetzt werden.

10. Verwendung nach Anspruch 8, dadurch gekennzeichnet, daß die Ester-Urethan-(Meth)-acrylsäurederivate in Beschichtungsmitteln für Zähne eingesetzt werden.

11. Verwendung nach Anspruch 8, dadurch gekennzeichnet, daß die Ester-Urethan-(Meth)Acrylsäure-Derivate für die Herstellung von Kunststoffzähnen eingesetzt werden.

12. Verwendung von Ester-Urethan-(Meth)-acrylsäurederivaten der Formel 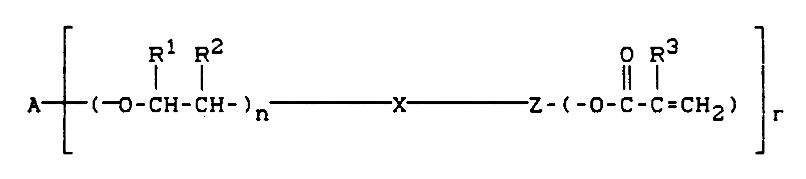 in der
A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
R¹ und R² gleich sind und Wasserstoff oder verschieden sind und Wasserstoff und Methyl bedeuten,
n für jede von A ausgehende Kette unabhängig eine Zahl von 0 bis 5 bedeutet,
X für eine Gruppe 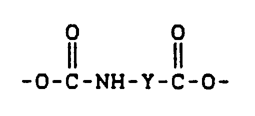 in der
Y einen zweiwertigen (cyclo)aliphatischen Rest mit 2 bis 15 Kohlenstoffatomen bedeutet, der gegebenenfalls Ester-, Ether- oder Urethangruppen enthält kann,
steht,
Z einen zweiwertiger geradkettiger oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acrylatreste substituiert sein kann, bedeutet, und
R³ für jede von A ausgehende Kette unabhängig Wasserstoff oder Methyl bedeutet,
zur Herstellung von Dentalwerkstoffen.

13. Dentalwerkstoffe, dadurch gekennzeichnet, daß sie Urethangruppen enthaltende (Meth)-acrylsäurederivate der Formel 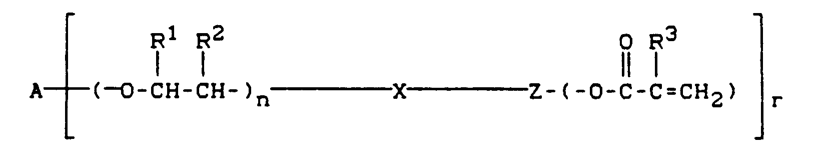 in der
A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
R¹ und R² gleich sind und Wasserstoff oder verschieden sind und Wasserstof und Methyl bedeuten,
n für jede von A ausgehende Kette unabhängig eine Zahl von 0 bis 5 bedeutet,
X für die Gruppe 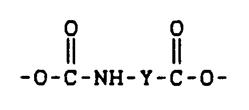 in der
Y einen zweiwertigen (cyclo)aliphatischen Rest mit 2 bis 15 Kohlenstoffatomen bedeutet, der gegebenenfalls Ester-, Ether- oder Urethangruppen enthälten kann,
steht,
Z einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acrylatrest substituiert sein kann, bedeutet, und
R³ für jede von A ausgehende Kette unabhängig Wasserstoff oder Methyl bedeutet,
enthalten.

14. Dentalwerkstoffe nach Anspruch 13, dadurch gekennzeichnet, daß sie neben Ester-Urethan-(Meth)-acrylsäurederivaten Comonomere enthalten.

15. Dentalwerkstoffe nach den Ansprüchen 13 bis 14, dadurch gekennzeichnet, daß sie neben Ester-Urethan(Meth)-acrylsäurederivaten und Comonomeren an sich bekannte Additive und gegebenenfalls Füllstoffe enthalten.

## Patentansprüche (Patentansprüche für folgende(n) Vertragsstaat(en): ES)

1. Verfahren zur Herstellung von polyfunktionellen Ester-Urethan-(Meth)-acrylsäurederivaten der Formel (I) 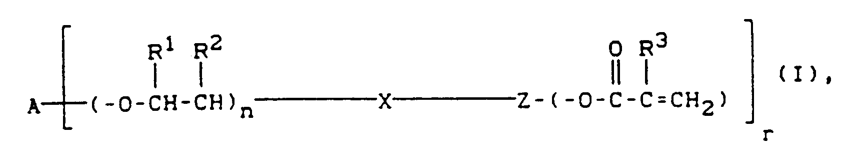 in der
A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,
r für die Anzahl der von A ausgehenden Ketten steht und eine Zahl von 2 bis 6 bedeutet,
R¹ und R² gleich sind und Wasserstoff oder verschieden sind und Wasserstoff und Methyl bedeuten,
n für jede von A ausgehende Kette unabhängig eine Zahl von 0 bis 5 bedeutet,
X für die Gruppe 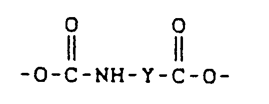 in der
Y einen zweiwertigen (cyclo)aliphatischen Rest mit 2 bis 15 Kohlenstoffatomen bedeutet, der gegebenenfalls Ester-, Ether- oder Urethangruppen enthalten kann,
steht,
Z einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 (Meth)-acrylatreste substituiert sein kann, bedeutet, und
R³ für jede von A ausgehende Kette unabhängig Wasserstoff oder Methyl bedeutet,
dadurch gekennzeichnet, daß man den Trialkylsilylether eines Polyols der Formel (II) 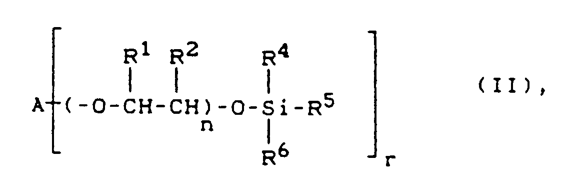 in der
A, R¹, R², n und r die obengenannte Bedeutung haben,
und
R⁴ bis R⁶ für einen Alkylrest mit 1 bis 4 Kohlenstoffatomen stehen,
mit einem Isocyanatocarbonsäurechlorid der Formel (III) 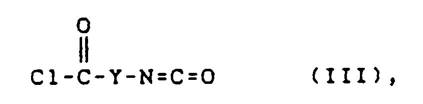 in der
Y die obengenannte Bedeutung hat,
im Molverhältnis von etwa 1:1, gegebenenfalls in einem inerten Lösungsmittel umsetzt und dann den entstandenen Isocyanato-ester mit einem Hydroxyalkyl(meth)acrylsäureester der Formel (IV) 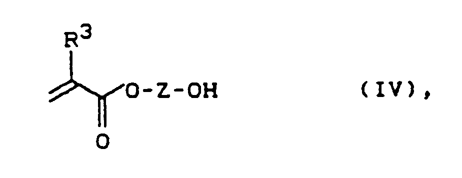 in der
R³ und Z die obengenannte Bedeutung haben,
im Molverhältnis von OH-Gruppen zu NCO-Gruppen von 0,98:1 bis 1,05:1 umsetzt.

2. Verfahren nach Anspruch 1, dadurch gekennzeichnet, daß man die Umsetzung des Silylethers mit dem Isocyanatocarbonsäurechlorid im Temperaturbereich von 80 bis 150°C durchführt.

3. Verfahren nach Anspruch 1, dadurch gekennnzeichnet, daß man die Umsetzung des Isocyanatoesters mit der Hydroxyverbindung im Temperaturbereich von 0 bis 100°C durchführt.

## Claims (Claims for the following Contracting State(s): AT, BE, CH, DE, FR, GB, IT, LI, NL, SE)

1. Polyfunctional ester-urethane derivatives of (meth)acrylic acid of the formula (I) 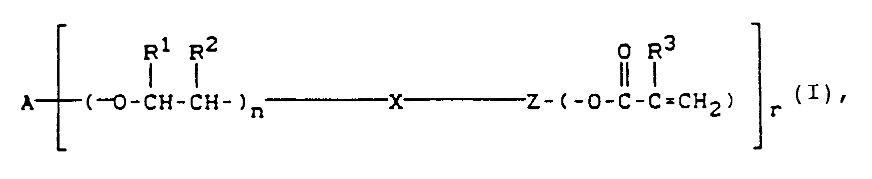 in which
A is a straight-chain or branched aliphatic radical which has 2 to 20 carbon atoms and optionally contains 1 to 3 oxygen bridges, an aromatic radical with 6 to 24 carbon atoms, an araliphatic radical with 7 to 26 carbon atoms or a cycloaliphatic radical with 6 to 26 carbon atoms,
r represents the number of chains starting from A and denotes a number from 2 to 6,
R¹ and R² are identical and denote hydrogen or are different and denote hydrogen and methyl,
n denotes a number from 0 to 5 independently for each chain starting from A,
X represents the group 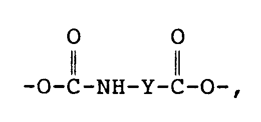 in which
Y denotes a divalent (cyclo)aliphatic radical which has 2 to 15 carbon atoms and optionally contains ester, ether or urethane groups,
Z denotes a divalent straight-chain or branched aliphatic hydrocarbon radical which has 3 to 15 carbon atoms and can optionally contain 1 to 3 oxygen bridges and can optionally be substituted by 1 to 4 additional (meth)-acrylate radicals and
R³ denotes hydrogen or methyl independently for each chain starting from A.

2. Polyfunctional ester-urethane derivatives of (meth)acrylic acid according to Claim 1,
wherein
A is a straight-chain or branched aliphatic radical which has 3 to 12 carbon atoms and optionally contains 1 to 3 oxygen bridges, an aromatic radical with 6 to 14 carbon atoms, an araliphatic radical with 7 to 26 carbon atoms or a cycloaliphatic radical with 6 to 14 carbon atoms,
r represents the number of chains starting from A and denotes a number from 2 to 6,
R¹ and R² are identical and denote hydrogen or are different and denote hydrogen and methyl,
n denotes a number from 0 to 5 independently for each chain starting from A,
X represents the group 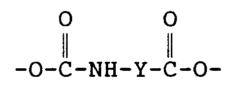 in which
Y denotes an alkylene radical with 2 to 10 carbon atoms,
Z denotes a divalent straight-chain or branched aliphatic hydrocarbon which has 3 to 10 carbon atoms and can optionally contain 1 or 2 oxygen bridge, and can optionally be substituted by 1 or 2 (meth)-acrylate radicals and
R³ denotes hydrogen or methyl independently for each chain starting from A.

3. Polyfunctional ester-urethane derivatives of (meth)acrylic acid according to Claims 1 and 2,
wherein
A represents the 2,2-bismethylene-butan-1-yl radical, propane-1,2,3-triyl radical, 2,2-bismethylenepropane-1,3-diyl radical or 3(4),8(9)-bismethylene-tricyclo[5.2.1.0^{2.6}]decaneradical,
r represents the number of chains starting from A and denotes the number 3 or 4,
R¹ and R² are identical and denote hydrogen or are different and denote hydrogen and methyl,
n denotes a number from 0 to 5 independently for each chain starting from A,
X denotes the group 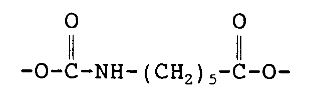
Z denotes a divalent straight-chain or branched aliphatic hydrocarbon radical which has 3 to 10 carbon atoms and can optionally contain 1 oxygen bridge and can optionally be substituted by 1 (meth)-acrylate radical and
R³ denotes hydrogen or methyl independently for each chain starting from A.

4. Process for the preparation of polyfunctional ester-urethane derivatives of (meth)-acrylic acid of the formula (I) 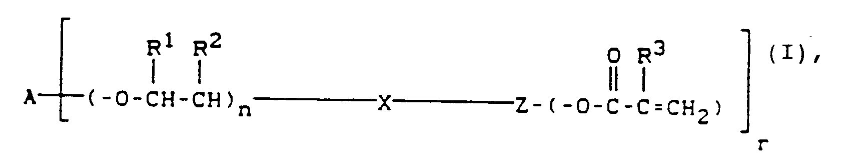 in which
A is a straight-chain or branched aliphatic radical which has 2 to 20 carbon atoms and optionally contains 1 to 3 oxygen bridges, an aromatic radical with 6 to 24 carbon atoms, an araliphatic radical with 7 to 26 carbon atoms or a cycloaliphatic radical with 6 to 26 carbon atoms,
r represents the number of chains starting from A and denotes a number from 2 to 6,
R¹ and R² are identical and denote hydrogen or are different and denote hydrogen and methyl,
n denotes a number from 0 to 5 independently for each chain starting from A,
X represents the group 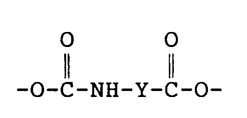 in which
Y denotes a divalent (cyclo)aliphatic radical which has 2 to 15 carbon atoms and can optionally contain ester, ether or urethane groups,
Z denotes a divalent straight-chain or branched aliphatic hydrocarbon radical which has 3 to 15 carbon atoms and can optionally contain 1 to 3 oxygen bridges and can optionally be substituted by 1 to 4 (meth)-acrylate radicals and
R³ denotes hydrogen or methyl independently for each chain starting from A,
characterised in that the trialkylsilyl ether of a polyol of the formula (II) 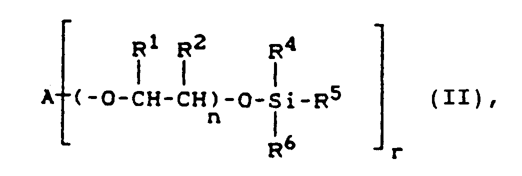 in which
A, R¹, R², n and r have the abovementioned meaning
and
R⁴ to R⁶ represent an alkyl radical with 1 to 4 carbon atoms,
is reacted with an isocyanatocarboxylic acid chloride of the formula (III) 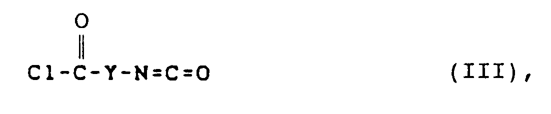 in which
Y has the abovementioned meaning,
in a molar ratio of about 1:1, if appropriate in an inert solvent, and the isocyanato ester formed is then reacted with a (meth)acrylic acid hydroxyalkyl ester of the formula (IV) 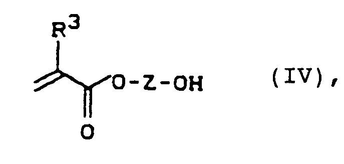 in which
R³ and Z have the abovementioned meaning,
in a molar ratio of OH groups to NCO groups of 0.98:1 to 1.05:1.

5. Process according to Claim 4, characterised in that the reaction of the silyl ether with the isocyanatocarboxylic acid chloride is carried out in the temperature range from 80 to 150°C.

6. Process according to Claims 4 and 5, characterised in that the reaction of the isocyanato ester with the hydroxyl compound is carried out in the temperature range from 0 to 100°C.

7. Polymer of ester-urethane derivatives of (meth)acrylic acid of the formula 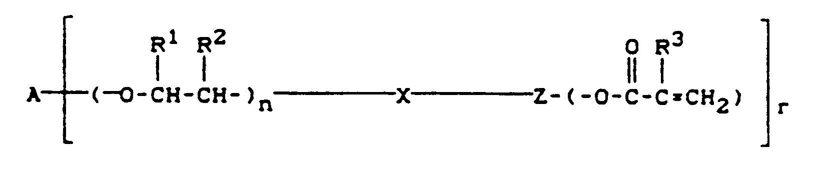 in which
A is a straight-chain or branched aliphatic radical which has 2 to 20 carbon atoms and optionally contains 1 to 3 oxygen bridges, an aromatic radical with 6 to 24 carbon atoms, an araliphatic radical with 7 to 26 carbon atoms or a cycloaliphatic radical with 6 to 26 carbon atoms,
r represents the number of chains starting from A and denotes a number from 2 to 6,
R¹ and R² are identical and denote hydrogen or are different and denote hydrogen and methyl,
n denotes a number from 0 to 5 independently for each chain starting from A,
X represents the group 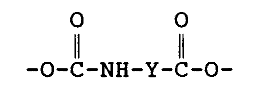 in which
Y denotes a divalent (cyclo)aliphatic radical which has 2 to 15 carbon atoms and optionally contains ester, ether or urethane groups,
Z denotes a divalent straight-chain or branched aliphatic hydrocarbon radical which has 3 to 15 carbon atoms and can optionally contain 1 to 3 oxygen bridges and can optionally be substituted by 1 to 4 (meth)-acrylate radicals and
R³ denotes hydrogen or methyl independently for each chain starting from A.

8. Use of ester-urethane derivatives of (meth)-acrylic acid of the formula 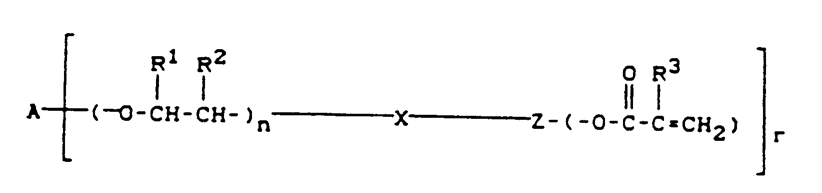 in which
A is a straight-chain or branched aliphatic radical which has 2 to 20 carbon atoms and optionally contains 1 to 3 oxygen bridges, an aromatic radical with 6 to 24 carbon atoms, an araliphatic radical with 7 to 26 carbon atoms or a cycloaliphatic radical with 6 to 26 carbon atoms,
r represents the number of chains starting from A and denotes a number from 2 to 6,
R¹ and R² are identical and denote hydrogen or are different and denote hydrogen and methyl,
n denotes a number from 0 to 5 independently for each chain starting from A,
X represents the group 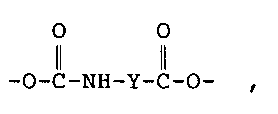 in which
Y denotes a divalent (cyclo)aliphatic radical which has 2 to 15 carbon atoms and can optionally contain ester, or urethane groups,
Z denotes a divalent straight-chain or branched aliphatic hydrocarbon radical which has 3 to 15 carbon atoms and can optionally contain 1 to 3 oxygen bridges and can optionally be substituted by 1 to 4 additional (meth)-acrylate radicals and
R³ denotes hydrogen or methyl independently for each chain starting from A,
in dental materials.

9. Use according to Claim 8, characterised in that the ester-urethane derivatives of (meth)-acrylic acid are used in dental filling compositions.

10. Use according to Claim 8, characterised in that the ester-urethane derivatives of (meth)-acrylic acid are used in coating agents for teeth.

11. Use according to Claim 8, characterised in that the ester-urethane derivatives of (meth)acrylic acid are used for the production of teeth of plastic.

12. Use of ester-urethane derivatives of (meth)-acrylic acid of the formula 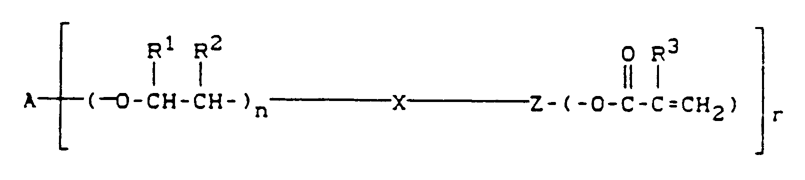 in which
A is a straight-chain or branched aliphatic radical which has 2 to 20 carbon atoms and optionally contains 1 to 3 oxygen bridges, an aromatic radical with 6 to 24 carbon atoms, an araliphatic radical with 7 to 26 carbon atoms or a cycloaliphatic radical with 6 to 26 carbon atoms,
r represents the number of chains starting from A and denotes a number from 2 to 6,
R¹ and R² are identical and denote hydrogen or are different and denote hydrogen and methyl,
n denotes a number from 0 to 5 independently for each chain starting from A,
X represents the group 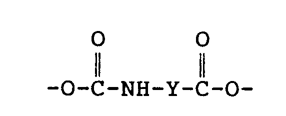 in which
Y denotes a divalent (cyclo)aliphatic radical which has 2 to 15 carbon atoms and can optionally contain ester, ether or urethane groups,
Z denotes a divalent straight-chain or branched aliphatic hydrocarbon radical which has 3 to 15 carbon atoms and can optionally contain 1 to 3 oxygen bridges and can optionally be substituted by 1 to 4 additional (meth)-acrylate radicals and
R³ denotes hydrogen or methyl independently for each chain starting from A,
for the preparation of dental materials.

13. Dental materials, characterised in that they contain (meth)-acrylic acid derivatives containing urethane groups, of the formula 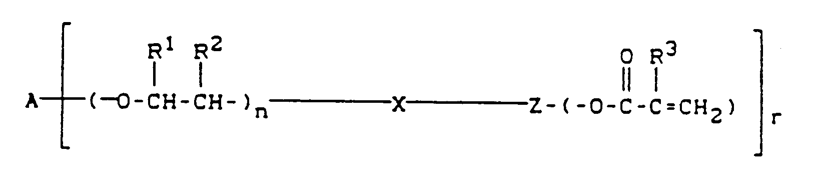 in which
A is a straight-chain or branched aliphatic radical which has 2 to 20 carbon atoms and optionally contains 1 to 3 oxygen bridges, an aromatic radical with 6 to 24 carbon atoms, an araliphatic radical with 7 to 26 carbon atoms or a cycloaliphatic radical with 6 to 26 carbon atoms,
r represents the number of chains starting from A and denotes a number from 2 to 6,
R¹ and R² are identical and denote hydrogen or are different and denote hydrogen and methyl,
n denotes a number from 0 to 5 independently for each chain starting from A,
X represents the group 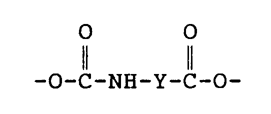 in which
Y denotes a divalent (cyclo)aliphatic radical which has 2 to 15 carbon atoms and can optionally contain ester, ether or urethane groups,
Z denotes a divalent straight-chain or branched aliphatic hydrocarbon radical which has 3 to 15 carbon atoms and can optionally contain 1 to 3 oxygen bridges and can optionally be substituted by 1 to 4 (meth)-acrylate radicals and
R³ denotes hydrogen or methyl independently for each chain starting from A.

14. Dental materials according to Claim 13, characterised in that they contain comonomers in addition to ester-urethane derivatives of (meth)-acrylic acid.

15. Dental materials according to Claims 13 and 14, characterised in that they contain additives and if appropriate fillers which are known per se, in addition to ester-urethane derivatives of (meth)acrylic acid and comonomers.

## Claims (Claims for the following Contracting State(s): ES)

1. Process for the preparation of polyfunctional ester-urethane derivatives of (meth)-acrylic acid of the formula (I) 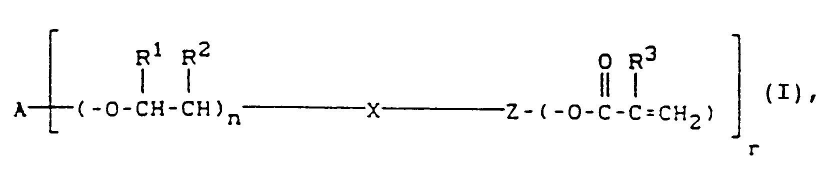 in which
A is a straight-chain or branched aliphatic radical which has 2 to 20 carbon atoms and optionally contains 1 to 3 oxygen bridges, an aromatic radical with 6 to 24 carbon atoms, an araliphatic radical with 7 to 26 carbon atoms or a cycloaliphatic radical with 6 to 26 carbon atoms,
r represents the number of chains starting from A and denotes a number from 2 to 6,
R¹ and R² are identical and denote hydrogen or are different and denote hydrogen and methyl,
n denotes a number from 0 to 5 independently for each chain starting from A,
X represents the group 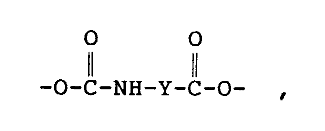 in which
Y denotes a divalent (cyclo)aliphatic radical which has 2 to 15 carbon atoms and can optionally contain ester, ether or urethane groups,
Z denotes a divalent straight-chain or branched aliphatic hydrocarbon radical which has 3 to 15 carbon atoms and can optionally contain 1 to 3 oxygen bridges and can optionally be substituted by 1 to 4 (meth)-acrylate radicals and
R³ denotes hydrogen or methyl independently for each chain starting from A,
characterised in that the trialkylsilyl ether of a polyol of the formula (II) 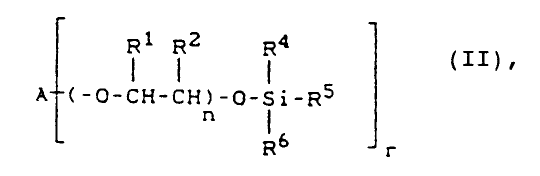 in which
A, R¹, R², n and r have the abovementioned meaning
and
R⁴ to R⁶ represent an alkyl radical with 1 to 4 carbon atoms,
is reacted with an isocyanatocarboxylic acid chloride of the formula (III) 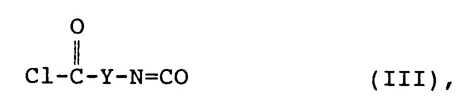 in which
Y has the abovementioned meaning,
in a molar ratio of about 1:1, if appropriate in an inert solvent, and the isocyanato ester formed is than reacted with a (meth)acrylic acid hydroxyalkyl ester of the formula (IV) 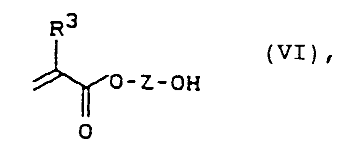 in which
R³ and Z have the abovementioned meaning,
in a molar ratio of OH groups to NCO groups of 0.98: to 1.05:1.

2. Process according to Claim 1, characterised in that the reaction of the silyl ether with the isocyanatocarboxylic acid chloride is carried out in the temperature range from 80 to 150°C.

3. Process according to Claim 1, characterised in that the reaction of the isocyanato ester with the hydroxyl compound is carried out in the temperature range from 0 to 100°C.

## Revendications (Revendications pour l'(les) Etat(s) contractant(s) suivant(s): AT, BE, CH, DE, FR, GB, IT, LI, NL, SE)

1. Dérivés polyfonctionnels du type ester-uréthanne d'acide (méth)acrylique de formule (I) 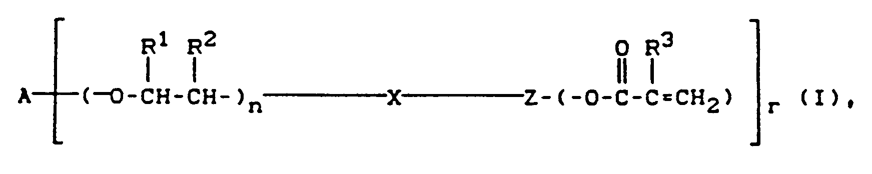 dans laquelle
A est un reste aliphatique de 2 à 20 atomes de carbone à chaîne droite ou ramifiée, contenant éventuellement 1 à 3 ponts oxygène, un reste aromatique de 6 à 24 atomes de carbone, un reste araliphatique ayant 7 à 26 atomes de carbone ou un reste cycloaliphatique de 6 à 26 atomes de carbone,
r représente le nombre de chaînes partant de A et désigne un nombre de 2 à 6,
R¹ et R² sont identiques et représentent l'hydrogène ou sont différents et représentent l'hydrogène et le groupe méthyle,
n représente, indépendamment, pour chaque chaîne partant de A, un nombre de 0 à 5,
X représente le groupe 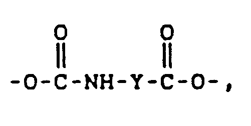 dans lequel
Y désigne un reste (cyclo)aliphatique divalent ayant 2 à 15 atomes de carbone, qui contient, le cas échéant, des groupes ester, éther ou uréthanne,
Z est un reste divalent d'hydrocarbure aliphatique à chaîne droite ou ramifiée ayant 3 à 15 atomes de carbone, qui peut comporter éventuellement 1 à 3 ponts oxygène et qui peut être substitué, le cas échéant, par 1 à 4 restes (méth)acrylate supplémentaires, et
R³ désigne, indépendamment, l'hydrogène ou le groupe méthyle pour chaque chaîne partant de A.

2. Dérivés polyfonctionnels du type ester-uréthanne d'acide (méth)acrylique suivant la revendication 1,
dans lesquels
A est un reste aliphatique à chaîne droite ou ramifiée de 3 à 12 atomes de carbone, contenant, le cas échéant, 1 à 3 ponts oxygène, un reste aromatique de 6 à 14 atomes de carbone, un reste araliphatique de 7 à 26 atomes de carbone ou un reste cycloaliphatique de 6 à 14 atomes de carbone,
r est le nombre de chaînes partant de A et désigne un nombre de 2 à 6,
R¹ et R² sont identiques et représentent l'hydrogène ou sont différents et représentent l'hydrogène et le groupe méthyle,
n pour chaque chaîne partant A, désigne, indépendamment, un nombre de 0 à 5,
X est le groupe 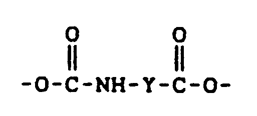 dans lequel
Y est un reste alkylène ayant 2 à 10 atomes de carbone,
Z est un hydrocarbure aliphatique divalent à chaîne droite ou ramifiée ayant 3 à 10 atomes de carbone, qui peut contenir éventuellement 1 à 2 ponts oxygène et qui peut être substitué, le cas échéant, par 1 ou 2 restes (méth)acrylate, et
R³ désigne, indépendamment, l'hydrogène ou le groupe méthyle pour chaque chaîne partant de A.

3. Dérivés polyfonctionnels du type ester-uréthanne d'acide (méth)acrylique suivant les revendications 1 et 2,
dans lesquels
A représente le reste 2,2-bisméthylène-butane-1-yle, le reste propane-1,2,3-triyle, le reste 2,2-bisméthylènepropane-1,3-diyle ou le reste 3(4),8(9)-bisméthylène-tricyclo[5.2.1.0^{2,6}]décane,
r représente le nombre de chaînes partant de A et désigne le nombre 3 ou 4,
R¹ et R² sont identiques et représentent l'hydrogène ou sont différents et représentent l'hydrogène et le groupe méthyle,
n désigne, indépendamment, pour chaque chaîne partant de A, un nombre de 0 à 5,
X est le groupe 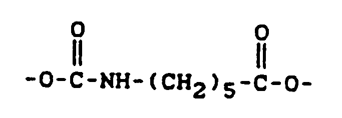
Z est un reste d'hydrocarbure aliphatique divalent à chaîne droite ou ramifiée ayant 3 à 10 atomes de carbone, qui comporte, le cas échéant, 1 pont oxygène et qui peut être substitué éventuellement par un reste (méth)acrylate, et
R³ désigne, indépendamment, l'hydrogène ou le groupe méthyle pour chaque chaîne partant de A.

4. Procédé de production de dérivés polyfonctionnels du type ester-uréthanne d'acide (méth)acrylique de formule (I) 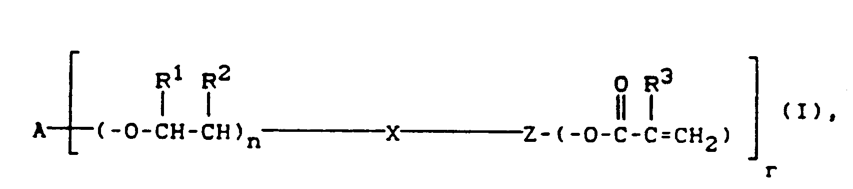 dans laquelle
A est un reste aliphatique de 2 à 20 atomes de carbone à chaîne droite ou ramifiée, contenant éventuellement 1 à 3 ponts oxygène, un reste aromatique de 6 à 24 atomes de carbone, un reste araliphatique ayant 7 à 26 atomes de carbone ou un reste cycloaliphatique de 6 à 26 atomes de carbone,
r représente le nombre de chaînes partant de A et désigne un nombre de 2 à 6,
R¹ et R² sont identiques et représentent l'hydrogène ou sont différents et représentent l'hydrogène et le groupe méthyle,
n représente, indépendamment, pour chaque chaîne partant de A, un nombre de 0 à 5,
X représente le groupe 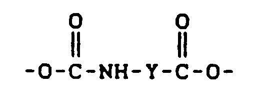 dans lequel
Y désigne un reste (cyclo)aliphatique divalent ayant 2 à 15 atomes de carbone, qui contient, le cas échéant, des groupes ester, éther ou uréthanne,
Z est un reste divalent d'hydrocarbure aliphatique à chaîne droite ou ramifiée ayant 3 à 15 atomes de carbone, qui peut comporter éventuellement 1 à 3 ponts oxygène et qui peut être substitué, le cas échéant, par 1 à 4 restes (méth)acrylate supplémentaires, et
R³ désigne, indépendamment, l'hydrogène ou le groupe méthyle pour chaque chaîne partant de A,
caractérisé en ce qu'on fait réagir l'éther de trialkylsilyle d'un polyol de formule (II) 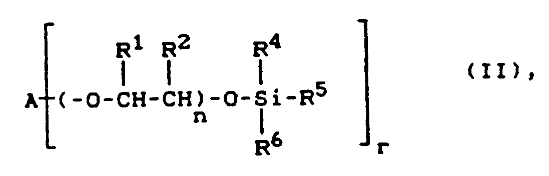 dans laquelle
A, R¹, R², n et r ont la définition indiquée ci-dessus,
et
R⁴ à R⁶ représentent un reste alkyle de 1 à 4 atomes de carbone,
avec un chlorure d'acide isocyanatocarboxylique de formule (III) 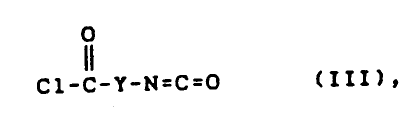 dans laquelle
Y a la définition indiquée ci-dessus,
dans le rapport molaire d'environ 1:1, le cas échéant dans un solvant inerte, puis on fait réagir l'isocyanato-ester correspondant avec un ester hydroxyalkylique d'acide (méth)acrylique de formule (IV) 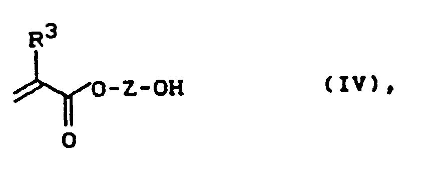 dans laquelle
R³ et Z ont la définition indiquée ci-dessus,
dans le rapport molaire des groupes OH aux groupes NCO de 0,98:1 à 1,05:1.

5. Procédé suivant la revendication 4, caractérisé en ce qu'on conduit la réaction de l'éther de silyle avec le chlorure d'acide isocyanatocarboxylique dans la plage de températures de 80 à 150°C.

6. Procédé suivant les revendications 4 et 5, caractérisé en ce qu'on conduit la réaction de l'isocyanatoester avec le composé hydroxylique dans la plage de températures de 0 à 100°C.

7. Polymère obtenu à partir de dérivés du type ester-uréthanne d'acide (méth)acrylique de formule 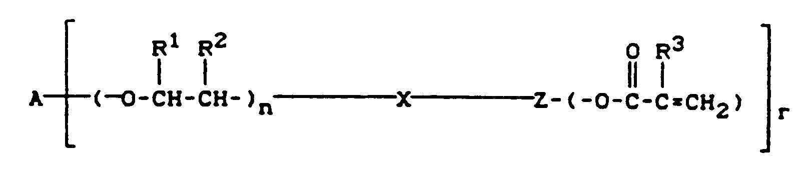 dans laquelle
A est un reste aliphatique de 2 à 20 atomes de carbone à chaîne droite ou ramifiée, contenant éventuellement 1 à 3 ponts oxygène, un reste aromatique de 6 à 24 atomes de carbone, un reste araliphatique ayant 7 à 26 atomes de carbone ou un reste cycloaliphatique de 6 à 26 atomes de carbone,
r représente le nombre de chaînes partant de A et désigne un nombre de 2 à 6,
R¹ et R² sont identiques et représentent l'hydrogène ou sont différents et représentent l'hydrogène et le groupe méthyle,
n représente, indépendamment, pour chaque chaîne partant de A, un nombre de 0 à 5,
X représente le groupe 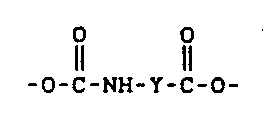 dans lequel
Y désigne un reste (cyclo)aliphatique divalent ayant 2 à 15 atomes de carbone, qui contient, le cas échéant, des groupes ester, éther ou uréthanne,
Z est un reste divalent d'hydrocarbure aliphatique à chaîne droite ou ramifiée ayant 3 à 15 atomes de carbone, qui peut comporter éventuellement 1 à 3 ponts oxygène et qui peut être substitué, le cas échéant, par 1 à 4 restes (méth)acrylate supplémentaires, et
R³ désigne, indépendamment, l'hydrogène ou le groupe méthyle pour chaque chaîne partant de A.

8. Utilisation de dérivés du type ester-uréthanne d'acide (méth)acrylique de formule 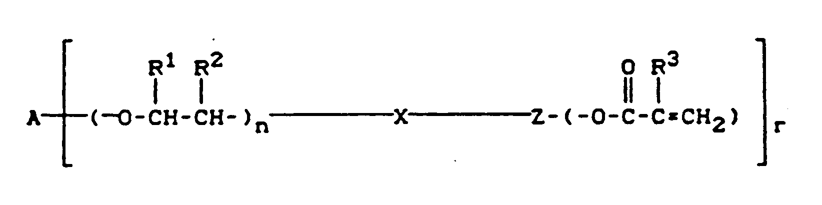 dans laquelle
A est un reste aliphatique de 2 à 20 atomes de carbone à chaîne droite ou ramifiée, contenant éventuellement 1 à 3 ponts oxygène, un reste aromatique de 6 à 24 atomes de carbone, un reste araliphatique ayant 7 à 26 atomes de carbone ou un reste cycloaliphatique de 6 à 26 atomes de carbone,
r représente le nombre de chaînes partant de A et désigne un nombre de 2 à 6,
R¹ et R² sont identiques et représentent l'hydrogène ou sont différents et représentent l'hydrogène et le groupe méthyle,
n représente, indépendamment, pour chaque chaîne partant de A, un nombre de 0 à 5,
X représente le groupe 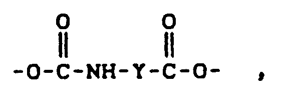 dans lequel
Y désigne un reste (cyclo)aliphatique divalent ayant 2 à 15 atomes de carbone, qui contient, le cas échéant, des groupes ester, éther ou uréthanne,
Z est un reste divalent d'hydrocarbure aliphatique à chaîne droite ou ramifiée ayant 3 à 15 atomes de carbone, qui peut comporter éventuellement 1 à 3 ponts oxygène et qui peut être substitué, le cas échéant, par 1 à 4 restes (méth)acrylate supplémentaires, et
R³ désigne, indépendamment, l'hydrogène ou le groupe méthyle pour chaque chaîne partant de A,
dans des matériaux dentaires.

9. Utilisation suivant la revendication 8, caractérisée en ce que les dérivés du type ester-uréthanne d'acide (méth)acrylique sont utilisés dans des mélanges d'obturation dentaire.

10. Utilisation suivant la revendication 8, caractérisée en ce que les dérivés du type ester-uréthanne d'acide (méth)acrylique sont utilisés dans des compositions de revêtement pour dents.

11. Utilisation suivant la revendication 8, caractérisée en ce que les dérivés du type ester-uréthanne d'acide (méth)acrylique sont utilisés pour la fabrication de dents artificielles.

12. Utilisation de dérivés du type ester-uréthanne d'acide (méth)acrylique de formule 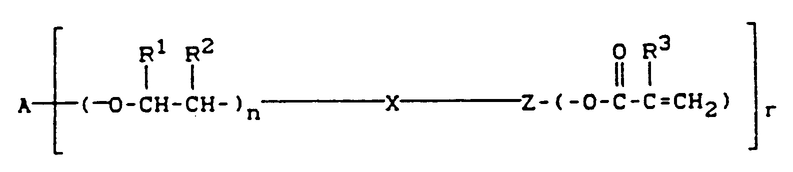 dans laquelle
A est un reste aliphatique de 2 à 20 atomes de carbone à chaîne droite ou ramifiée, contenant éventuellement 1 à 3 ponts oxygène, un reste aromatique de 6 à 24 atomes de carbone, un reste araliphatique ayant 7 à 26 atomes de carbone ou un reste cycloaliphatique de 6 à 26 atomes de carbone,
r représente le nombre de chaînes partant de A et désigne un nombre de 2 à 6,
R¹ et R² sont identiques et représentent l'hydrogène ou sont différents et représentent l'hydrogène et le groupe méthyle,
n représente, indépendamment, pour chaque chaîne partant de A, un nombre de 0 à 5,
X représente le groupe 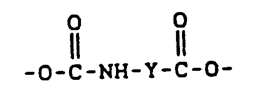 dans lequel
Y désigne un reste (cyclo)aliphatique divalent ayant 2 à 15 atomes de carbone, qui contient, le cas échéant, des groupes ester, éther ou uréthanne,
Z est un reste divalent d'hydrocarbure aliphatique à chaîne droite ou ramifiée ayant 3 à 15 atomes de carbone, qui peut comporter éventuellement 1 à 3 ponts oxygène et qui peut être substitué, le cas échéant, par 1 à 4 restes (méth)acrylate supplémentaires, et
R³ désigne, indépendamment, l'hydrogène ou le groupe méthyle pour chaque chaîne partant de A,
pour la production de matériaux dentaires.

13. Matériaux dentaires, caractérisés en ce qu'ils contiennent des dérivés d'acide (méth)acrylique porteurs de groupes uréthanne, de formule 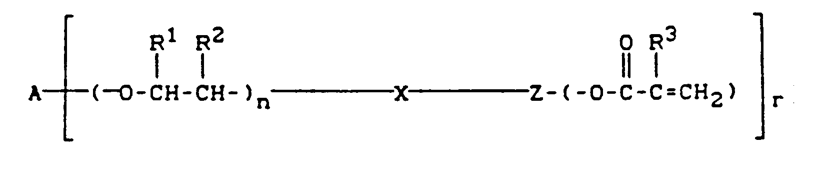 dans laquelle
A est un reste aliphatique de 2 à 20 atomes de carbone à chaîne droite ou ramifiée, contenant éventuellement 1 à 3 ponts oxygène, un reste aromatique de 6 à 24 atomes de carbone, un reste araliphatique ayant 7 à 26 atomes de carbone ou un reste cycloaliphatique de 6 à 26 atomes de carbone,
r représente le nombre de chaînes partant de A et désigne un nombre de 2 à 6,
R¹ et R² sont identiques et représentent l'hydrogène ou sont différents et représentent l'hydrogène et le groupe méthyle,
n représente, indépendamment, pour chaque chaîne partant de A, un nombre de 0 à 5,
X représente le groupe 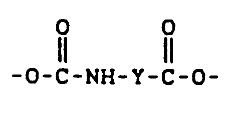 dans lequel
Y désigne un reste (cyclo)aliphatique divalent ayant 2 à 15 atomes de carbone, qui contient, le cas échéant, des groupes ester, éther ou uréthanne,
Z est un reste divalent d'hydrocarbure aliphatique à chaîne droite ou ramifiée ayant 3 à 15 atomes de carbone, qui peut comporter éventuellement 1 à 3 ponts oxygène et qui peut être substitué, le cas échéant, par 1 à 4 restes (méth)acrylate supplémentaires, et
R³ désigne, indépendamment, l'hydrogène ou le groupe méthyle pour chaque chaîne partant de A.

14. Matériaux dentaires suivant la revendication 13, caractérisés en ce qu'ils contiennent des comonomères à côté de dérivés du type ester-uréthanne d'acide (méth)acrylique.

15. Matériaux dentaires suivant les revendications 13 et 14, caractérisés en ce qu'ils contiennent, à côté de dérivés du type ester-uréthanne d'acide (méth)acrylique et de comonomères, des additifs connus et, le cas échéant, des charges.

## Revendications (Revendications pour l'(les) Etat(s) contractant(s) suivant(s): ES)

1. Procédé de production de dérivés polyfonctionnels du type ester-uréthanne d'acide (méth)acrylique de formule (I) 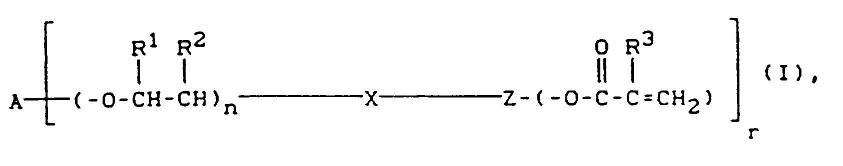 dans laquelle
A est un reste aliphatique de 2 à 20 atomes de carbone à chaîne droite ou ramifiée, contenant éventuellement 1 à 3 ponts oxygène, un reste aromatique de 6 à 24 atomes de carbone, un reste araliphatique ayant 7 à 26 atomes de carbone ou un reste cycloaliphatique de 6 à 26 atomes de carbone,
r représente le nombre de chaînes partant de A et désigne un nombre de 2 à 6,
R¹ et R² sont identiques et représentent l'hydrogène ou sont différents et représentent l'hydrogène et le groupe méthyle,
n représente, indépendamment, pour chaque chaîne partant de A, un nombre de 0 à 5,
X représente le groupe 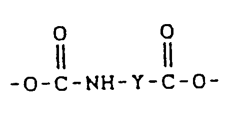 dans lequel
Y désigne un reste (cyclo)aliphatique divalent ayant 2 à 15 atomes de carbone, qui contient, le cas échéant, des groupes ester, éther ou uréthanne,
Z est un reste divalent d'hydrocarbure aliphatique à chaîne droite ou ramifiée ayant 3 à 15 atomes de carbone, qui peut comporter éventuellement 1 à 3 ponts oxygène et qui peut être substitué, le cas échéant, par 1 à 4 restes (méth)acrylate supplémentaires, et
R³ désigne, indépendamment, l'hydrogène ou le groupe méthyle pour chaque chaîne partant de A,
caractérisé en ce qu'on fait réagir l'éther de trialkylsilyle 'un polyol de formule (II) 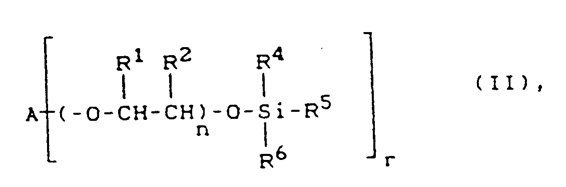 dans laquelle
A, R¹, R², n et r ont la définition indiquée ci-desus,
et
R⁴ à R⁶ représentent un reste alkyle de 1 à 4 atomes de carbone,
avec un chlorure d'acide isocyanatocarboxylique de formule (III) 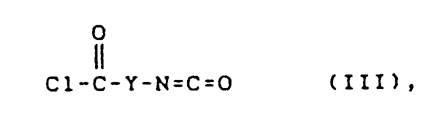 dans laquelle
Y a la définition indiquée ci-dessus,
dans le rapport molaire d'environ 1:1, le cas échéant dans un solvant inerte, puis on fait réagir l'isocyanato-ester correspondant avec un ester hydroxyalkylique d'acide (méth)acrylique de formule (IV) 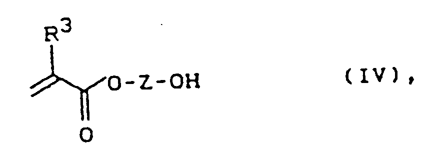 dans laquelle
R³ et Z ont la définition indiquée ci-dessus,
dans le rapport molaire des groupes OH aux groupes NCO de 0,98:1 à 1,05:1.

2. Procédé suivant la revendication 1, caractérisé en ce qu'on conduit la réaction de l'éther de silyle avec le chlorure d'acide isocyanatocarboxylique dans la plage de températures de 80 à 150°C.

3. Procédé suivant la revendication 1, caractérisé en ce qu'on conduit la réaction de l'isocyanato-ester avec le composé hydroxylique dans la plage de températures de 0 à 100°C.
